# EUROPÄISCHE PATENTANMELDUNG

(11) **EP 4 664 138 A1**
(43) Veröffentlichungstag der Anmeldung: **17.12.2025**
(21) Anmeldenummer: 24181398.9
(22) Anmeldetag: 11.06.2024
(51) Int. Cl.: G01R 33/54, A61B 6/46, A61B 6/00

(54) **BILDGEBUNGSVORRICHTUNG UND VERFAHREN ZUR BESTIMMUNG EINES BILDGEBUNGSPROTOKOLLS**

(71) Anmelder: Siemens Healthineers AG, 91301 Forchheim (DE)
(72) Erfinder: Rupp, Dominik, 90587 Tuchenbach (DE); Van de Stadt-Lagemaat, Miriam, 91056 Erlangen (DE); Mehler, Frank, 91077 Neunkirchen am Brand (DE)
(74) Vertreter: Siemens Healthineers Patent Attorneys

(57) **Zusammenfassung**

Die Erfindung betrifft ein Computer-implementiertes Verfahren zur Bestimmung eines Bildgebungsprotokolls zur Erfassung von Bilddaten einer Körperregion eines Patienten mittels einer Bildgebungsvorrichtung, umfassend die Schritte: Erfassen (S1) einer Information über die Körperregion, Durchführen (S2) einer ersten Bildgebungsuntersuchung in Abhängigkeit der Information über die Körperregion und Erfassen von Bilddaten der Körperregion, Bereitstellen (S3) der Bilddaten und einer Eingabeoption zur Anpassung eines Parameters eines Bildgebungsbereichs, Bereitstellen (S4) einer Hilfestellung zur Anpassung des Bildgebungsbereichs in Abhängigkeit der Information über die Körperregion, Erfassen (S5) eines angepassten Bildgebungsbereichs, und Bestimmen (S6) des Bildgebungsprotokolls für eine zweite Bildgebungsuntersuchung in Abhängigkeit des angepassten Bildgebungsbereichs. Die Erfindung betrifft ferner eine Bildgebungsvorrichtung zur Erfassung von Bilddaten einer Körperregion eines Patienten und ein Computerprogrammprodukt, welches direkt in eine Speichereinheit einer Recheneinheit (28) einer erfindungsgemäßen Bildgebungsvorrichtung ladbar ist, mit Programmcode-Mitteln, um ein erfindungsgemäßes Computer-implementiertes Verfahren auszuführen.

## Beschreibung

Unabhängig vom grammatikalischen Geschlecht eines bestimmten Begriffes sind Personen mit männlicher, weiblicher oder anderer Geschlechteridentität mit umfasst.

Die Planung eines Bildgebungsprotokolls zur Erfassung von Bilddaten einer diagnostisch relevanten Körperregion eines Patienten mittels einer Bildgebungsvorrichtung kann von patientenspezifischen Voraussetzungen und/oder nutzerspezifischen Eingaben bei einer Patientenregistrierung sowie einer Vorbereitung des Patienten für eine Bildgebungsuntersuchung abhängen. Beispielsweise sind bei der Planung des Bildgebungsprotokolls eine Lateralität der diagnostisch relevanten Körperregion (z. B. das Vorhandensein in einer linken oder rechten Körperhälfte), eine Unterteilung der Körperregion in relevante Abschnitte und/oder eine Orientierung der Körperregion in einem Bezugssystem für die Auswahl einer geeigneten Schichtausrichtung zu beachten. Insbesondere bei Bildgebungsprotokollen für die Zahn- oder Kieferregion des Patienten kann eine Spezifikation einer geeigneten Schichtausrichtung sowie eine Auswahl von relevanten Abschnitten aufgrund des symmetrischen Aufbaus der Zahnbögen (z. B. linker, rechter, oder frontaler Unterkiefer und/oder Oberkiefer) und der geschwungenen Form der Zahnbögen komplex sein. Daher müssen Nutzer der Bildgebungsvorrichtung aufwändig trainiert werden, um ein fehlerfreies Erfassen von Bilddaten der diagnostisch relevanten Körperregionen mittels einer Bildgebungsvorrichtung im klinischen Alltag zu gewährleisten.

Kommerziell verfügbare Bildgebungsvorrichtungen verfügen typischerweise über eine festgelegte Anleitung für die Schichtausrichtung auf Protokollebene. Solche festgelegten Anleitungen berücksichtigen jedoch selten die Lateralität der diagnostisch relevanten Körperregion und zeigen beispielsweise eine Schichtpositionierung für die linke Hüfte eines Patienten an, obwohl die rechte Hüfte des Patienten diagnostisch relevant ist. Zwar sollten festgelegte Anleitungen als Beispiele verstanden werden, aber die Nutzer der Bildgebungsvorrichtung müssen über eine ausreichende Ausbildung und Erfahrung verfügen, um Messungen für alle möglichen Anwendungsfälle sowie komplexe anatomische Strukturen zu planen.

In einigen Ausnahmefällen werden bei Vorliegen einer geringen Anzahl von eingesetzten Schichtausrichtungen verschiedene Parameter oder Szenarien für den Nutzer voreingestellt, wobei jede Schichtausrichtung mit einer entsprechenden Anleitung hinterlegt wird. Dies führt jedoch zu einer Duplikation von Anleitungen, Workflows und Bildgebungsprotokollen, welche nicht erwünscht ist.

Es ist daher eine Aufgabe der Erfindung, einen Prozess einer Bestimmung eines Bildgebungsprotokolls für eine Erfassung von Bilddaten einer Körperregion eines Patienten mittels einer Bildgebungsvorrichtung zu verbessern.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Das erfindungsgemäße Computer-implementierte Verfahren zur Bestimmung eines Bildgebungsprotokolls zur Erfassung von Bilddaten einer Körperregion eines Patienten mittels einer Bildgebungsvorrichtung umfasst die folgenden Schritte:
- Erfassen einer Information über die Körperregion,
- Durchführen einer ersten Bildgebungsuntersuchung in Abhängigkeit der Information über die Körperregion und Erfassen von Bilddaten der Körperregion,
- Bereitstellen der Bilddaten und einer Eingabeoption zur Anpassung eines Parameters eines Bildgebungsbereichs,
- Bereitstellen einer Hilfestellung zur Anpassung des Bildgebungsbereichs in Abhängigkeit der Information über die Körperregion,
- Erfassen eines angepassten Bildgebungsbereichs, und
- Bestimmen des Bildgebungsprotokolls in Abhängigkeit des angepassten Bildgebungsbereichs.

Eine Bildgebungsvorrichtung kann ein Gerät darstellen, welches dazu ausgebildet ist, Bilddaten eines Untersuchungsobjekts, insbesondere einer Körperregion oder eines Inneren eines Patienten, zu erfassen. Vorzugsweise ist eine Bildgebungsvorrichtung dazu ausgebildet, zweidimensionale und/oder dreidimensionale Bilddaten, insbesondere zeitabhängige dreidimensionale Bilddaten, des Untersuchungsobjekts aufzunehmen. Beispiele für Bildgebungsvorrichtungen sind Magnetresonanzgeräte, Röntgengeräte, Computertomografiegeräte, Einzelphotonen-Emissions-Computertomografen, Positron-Emissions-Tomografen, aber auch Mammografie-Geräte, Ultraschallgeräte und dergleichen. In einer bevorzugten Ausführungsform ist die Bildgebungsvorrichtung als ein Magnetresonanzgerät ausgestaltet.

Ein Bildgebungsprotokoll einer Bildgebungsuntersuchung kann durch einen oder mehrere Bildgebungsparameter charakterisiert sein. Beispiele für Bildgebungsparameter sind eine räumliche Auflösung, ein Kontrast, eine Schichtdicke, eine Abmessung eines Bildgebungsvolumens, eine Relaxationszeit, eine Echozeit und dergleichen. Ein Bildgebungsparameter kann eine beliebige bildrelevante Einstellung der Bildgebungsvorrichtung, aber auch einen Parameter eines Workflows der Bildgebungsuntersuchung, umfassen. Weiterhin können auch Parameter, welche einen Bildgebungsbereich definieren, als Bildgebungsparameter verstanden werden.

Eine Bildgebungsuntersuchung kann eine oder mehrere Gruppen von Bildgebungsparametern sowie eine oder mehrere Bildgebungssequenzen umfassen.

Ein Bildgebungsprotokoll kann einen Ablauf einer Bildgebungsuntersuchung einer Körperregion eines Patienten definieren. Beispielsweise kann das Durchführen einer Bildgebungsuntersuchung ein Durchführen einer oder mehrerer Bildgebungssequenzen umfassen. Bei dem Durchführen der Bildgebungssequenzen können Bilddaten der Körperregion des Patienten erfasst werden.

Das Bildgebungsprotokoll kann dazu angepasst sein, Bilddaten einer Körperregion zu erfassen. Die Körperregion kann beispielsweise eine Hüftregion, eine Schulterregion, eine Knieregion, eine Hirnregion, eine Augenregion oder eine beliebige andere anatomische Struktur umfassen. Es ist ebenso vorstellbar, dass die Körperregion eine Gewebestruktur oder ein Organ, wie z. B. ein Herz, eine Leber, eine Niere, oder dergleichen, umfasst.

Vorzugsweise umfasst das Bildgebungsprotokoll oder eine auf dem Bildgebungsprotokoll basierende Bildgebungsuntersuchung ein Ausführen einer oder mehrerer Bildgebungssequenzen, welche für eine Bildgebung einer Kieferregion, einer Zahnregion und/oder eines Zahns angepasst sind.

In einer bevorzugten Ausführungsform sind die erste Bildgebungsuntersuchung und/oder die zweite Bildgebungsuntersuchung Magnetresonanzuntersuchungen einer Kieferregion oder einer Zahnregion eines Patienten. Es ist vorstellbar, dass eine Bildgebungssequenz von zumindest einer Magnetresonanzuntersuchung eine sehr kurze Echozeit aufweist, um eine kurze T2-Relaxationszeit von Spins eines Dentins oder eines Zahnschmelzes eines Zahns zu kompensieren und diese Bereiche mit hoher Signalintensität in erfassten Bilddaten darzustellen. Sehr kurze Echozeiten können kleiner als 150 µs oder kleiner als 70 µs sein. Mögliche Bildgebungssequenzen stellen beispielsweise FLASH ("*fast low-angle shot*") oder UTE (*"ultra-short echo time*") Sequenzen dar. Es ist jedoch ebenso vorstellbar, dass Bildgebungssequenzen mit einer längeren Echozeit, wie z. B. einer TSE ("*turbo spin echo*") Sequenz, verwendet werden. Bei solchen Sequenzen kann ein Erfassen des Magnetresonanzsignals des Zahnschmelzes oder des Dentins vermieden werden. In Bilddaten solcher Bildgebungssequenzen lassen sich die Zähne durch einen Mangel an Signalintensität im Vergleich zu einem umliegenden Gewebe differenzieren.

Bilddaten können beliebige Daten darstellen, welcher mittels der Bildgebungsvorrichtung von der Körperregion des Patienten erfasst werden. Bilddaten können sowohl Rohdaten als auch Bilder, welche aus den Rohdaten abgeleitet werden, umfassen. Beispielsweise können die Bilddaten von einem Magnetresonanzgerät erfasste, digitalisierte Magnetresonanzsignale umfassen. Die Bilddaten können als komplexe Werte in einer k-Raum Matrix gespeichert sein. Vorzugsweise umfassen die Bilddaten jedoch auch Magnetresonanzbilder, welcher in Abhängigkeit der digitalisierten Magnetresonanzsignale rekonstruiert wurden.

Eine Information über die Körperregion eines Patienten kann eine beliebige Beschreibung eines Typs, einer Bezeichnung, einer Position und/oder einer Ausdehnung der Körperregion des Patienten umfassen. Die Information über die Körperregion kann ferner eine Selektion und/oder eine Identifikation einer Körperregion oder einer anatomischen Struktur aus einer Liste oder einer Datenbank von Körperregionen umfassen.

Vorzugsweise umfasst das Erfassen der Information über die Körperregion des Patienten ein Erfassen einer Eingabe eines Nutzers der Bildgebungsvorrichtung mittels einer geeigneten Eingabeschnittstelle oder Benutzerschnittstelle, wie z. B. einer Maus, einer Tastatur, eines Touchscreens und/oder einer Sprachschnittstelle. Das Erfassen der Information über die Körperregion des Patienten kann weiterhin ein Abrufen oder Empfangen von Daten, insbesondere von Patientendaten oder Patienteninformationen, von einer internen oder externen Speichereinheit und/oder einem medizinischen Informationssystem mittels einer Schnittstelle umfassen. Ein medizinisches Informationssystem kann beispielsweise ein radiologisches Informationssystem (RIS) oder ein Krankenhaus-Informationssystem ("*hospital information system*" HIS) darstellen.

Die Information über die Körperregion des Patienten kann manuell von einem Nutzer der Bildgebungsvorrichtung mittels einer Benutzerschnittstelle eingegeben werden. Insbesondere kann die Information über die Körperregion mittels einer Auswahl einer anatomischen Struktur oder eines Abschnitts einer anatomischen Struktur in Abhängigkeit einer mittels der Benutzerschnittstelle bereitgestellten Repräsentation der anatomischen Struktur durch den Nutzer der Bildgebungsvorrichtung ausgewählt werden. Es ist jedoch ebenso vorstellbar, dass die Bildgebungsvorrichtung eine Steuereinheit und/oder eine Recheneinheit aufweist, welche dazu ausgebildet sind, die Information über die Körperregion des Patienten in Abhängigkeit einer Patienteninformation mittels einer geeigneten Schnittstelle von dem medizinischen Informationssystem, einer Cloud und/oder einer lokalen Speichereinheit zu beziehen. Insbesondere kann die Information über die Körperregion mittels eines Algorithmus oder eines Bildverarbeitungsalgorithmus in Abhängigkeit der Bilddaten der ersten Bildgebungsuntersuchung und/oder der Patienteninformation ermittelt werden. Die Patienteninformation kann einen Befund oder eine Diagnose, aber auch ein Alter, ein Geschlecht, ein Gewicht oder eine beliebige andere medizinische oder demographische Information über den Patienten umfassen.

In einer bevorzugten Ausführungsform des erfindungsgemäßen Verfahrens umfasst das Erfassen der Information über die Körperregion des Patienten ein Erfassen eines Abschnitts einer Zahnregion, insbesondere eines Abschnitts eines oder mehrerer Zahnbögen, des Patienten.

Die erste Bildgebungsuntersuchung ist vorzugsweise eine Localizer-Messung. Eine Localizer-Messung kann als eine zeiteffiziente Bildgebungsuntersuchung verstanden werden, bei welcher Bilddaten, insbesondere Localizer-Bilddaten, der Körperregion des Patienten erfasst werden. Die Localizer-Messung kann Einschränkungen bezüglich einer Qualität und/oder einer räumlichen Auflösung der erfassten Bilddaten im Vergleich zu einer konventionellen Bildgebungsuntersuchung aufweisen. Vorzugsweise stellt die Localizer-Messung eine räumliche Auflösung bereit, welche für eine Detektion und/oder eine Identifizierung von anatomischen Strukturen, wie z. B. einem Zahn, einem Zahnbogen, einem Kieferknochen oder dergleichen, geeignet ist. Die erste Bildgebungsuntersuchung kann ferner eine Projektionsmessung umfassen. Eine Projektionsmessung kann eine Bildgebungsuntersuchung darstellen, bei welcher eine räumliche Kodierung in einer Raumrichtung entfällt. Die Bilddaten können somit ein zweidimensionales Projektionsbild eines dreidimensionalen Volumens der Körperregion des Patienten umfassen. Es ist weiterhin vorstellbar, dass die Bilddaten ein Bild einer vorangegangenen Bildgebungsuntersuchung, insbesondere einer vorangegangenen Magnetresonanzuntersuchung, umfassen.

In einer bevorzugten Ausführungsform des erfindungsgemäßen Verfahrens erfolgt das Erfassen von Bilddaten der Körperregion mittels eines Antennenelements oder einer Mehrzahl von Antennenelementen eines Magnetresonanzgerät. Die Antennenelemente können hierfür beispielsweise an einer Kieferregion und/oder in einer Mundhöhle des Patienten positioniert sein. Das Antennenelement oder die Mehrzahl von Antennenelementen können insbesondere dazu ausgelegt sein, Magnetresonanzsignale der Kieferregion zu empfangen und diese an eine Empfangseinheit des Magnetresonanzgeräts zu übermitteln.

Das Durchführen der ersten Bildgebungsuntersuchung kann in Abhängigkeit der Information über die Körperregion erfolgen. Es ist jedoch ebenso vorstellbar, dass das Durchführen der ersten Bildgebungsuntersuchung unabhängig von dem Erfassen der Information über die Körperregion des Patienten erfolgt (z. B. als unabhängige Localizer-Messung oder Übersichtsmessung). Das Durchführen der ersten Bildgebungsuntersuchung erlaubt ein Erfassen von Bilddaten der Körperregion des Patienten. Die Bilddaten können insbesondere Localizer-Bilddaten oder weitere Bilddaten nach einer unten beschriebenen Ausführungsform umfassen.

Ein Bereitstellen der Bilddaten der Körperregion des Patienten umfasst vorzugsweise zumindest ein Abspeichern der Bilddaten auf einer lokalen Speichereinheit der Bildgebungsvorrichtung, einer Netzwerk-Speichereinheit, eines medizinischen Informationssystems und/oder einer Cloud. Es ist weiterhin vorstellbar, dass das Bereitstellen der Bilddaten der Körperregion ein Ausgeben der Bilddaten mittels einer Ausgabeeinheit an einen Nutzer der Bildgebungsvorrichtung umfasst. Eine Ausgabeeinheit kann beispielsweise als ein Bildschirm, ein Monitor, ein Touchscreen, ein Projektor oder dergleichen ausgestaltet sein.

Ein Nutzer der Bildgebungsvorrichtung kann beispielsweise ein Mediziner, insbesondere ein Zahnmediziner, ein medizinisch-technischer Assistent oder ein Mitglied eines medizinischen Personals einer Praxis oder einer klinischen Einrichtung sein. Der Nutzer kann an einem Standort der Bildgebungsvorrichtung, aber auch an einem beliebigen anderen Ort positioniert sein. Beispielsweise kann der Nutzer in einer anderen Stadt, einer anderen Region und/oder einem anderen Land positioniert sein und ferngesteuert mit der Bildgebungsvorrichtung interagieren.

Es ist weiterhin vorstellbar, dass die Bilddaten der Körperregion des Patienten von einem Programm und/oder einem Algorithmus, welche dazu ausgebildet sind, einen Bildinhalt oder eine oder mehrere anatomische Strukturen zu kategorisieren, zu identifizieren und/oder zu klassifizieren, verarbeitet werden. Es ist insbesondere vorstellbar, dass das Programm und/oder der Algorithmus dazu ausgebildet sind, eine Hilfestellung zur Anpassung des Bildgebungsbereichs und/oder einen vorläufigen Bildgebungsbereich in Abhängigkeit der Information über die Körperregion und der Bilddaten zu ermitteln. In einer Ausführungsform des erfindungsgemäßen Verfahrens sind das Programm und/oder der Algorithmus dazu ausgebildet, die Hilfestellung zur Anpassung des Bildgebungsbereichs und/oder den vorläufigen Bildgebungsbereich in Abhängigkeit von Patienteninformationen zu ermitteln.

Das Bereitstellen der Eingabeoption zur Anpassung des Parameters eines Bildgebungsbereichs umfasst vorzugsweise ein Ausgeben der Eingabeoption mittels einer Ausgabeeinheit und/oder einer Benutzerschnittstelle. Beispielsweise kann die Eingabeoption zur Anpassung des Parameters des Bildgebungsbereichs mittels einer grafischen Benutzerschnittstelle, insbesondere eines Monitors oder eines Touchscreens, an den Nutzer ausgegeben werden. Die Eingabeoption zur Anpassung des Parameters des Bildgebungsbereichs kann eine Eingabemaske umfassen, welche es dem Nutzer ermöglicht, einen Parameter des Bildgebungsbereichs zu ändern oder anzupassen. Ein Parameter des Bildgebungsbereichs kann beispielsweise eine räumliche Position, eine Ausrichtung, eine Dimension, und/oder eine Form des Bildgebungsbereichs definieren.

Der Bildgebungsbereich kann als ein Messvolumen, ein Field-of-View, ein Sichtfenster oder eine Schichtausrichtung verstanden werden. Insbesondere kann der Bildgebungsbereich ein Volumen innerhalb eines Patientenaufnahmebereichs der Bildgebungsvorrichtung definieren, aus welchem Bilddaten mittels einer Bildgebungsuntersuchung erfasst werden. Vorzugsweise ist zumindest ein Abschnitt der Körperregion des Patienten für eine Bildgebungsuntersuchung in einem sogenannten Bildgebungsvolumen der Bildgebungsvorrichtung positioniert. Der Bildgebungsbereich kann als ein Volumen innerhalb des Bildgebungsvolumens der Bildgebungsvorrichtung verstanden werden, auf welches das Erfassen von Bilddaten beschränkt ist.

In einer bevorzugten Ausführungsform ist die Bildgebungsvorrichtung als ein Magnetresonanzgerät ausgestaltet. Ein Bildgebungsvolumen kann ein Volumen mit einer höchsten Homogenität eines Magnetfelds, insbesondere ein Isozentrum, des Magnetresonanzgeräts darstellen.

Das Bereitstellen Hilfestellung zur Anpassung des Bildgebungsbereichs umfasst vorzugsweise ein Ausgeben einer Information über eine gewünschte oder ideale Einstellung des Bildgebungsbereichs mittels einer Ausgabeeinheit oder einer grafischen Benutzerschnittstelle gemäß einer oben beschriebenen Ausführungsform. Beispielsweise kann die Hilfestellung zur Anpassung des Bildgebungsbereichs auf einem Monitor für den Nutzer dargestellt werden.

Die Hilfestellung zur Anpassung des Bildgebungsbereichs wird in Abhängigkeit der Information über die Körperregion bereitstellt.

Das Bereitstellen der Hilfestellung zur Anpassung des Bildgebungsbereichs kann ein Auswählen einer Information über eine gewünschte oder ideale Einstellung des Bildgebungsbereichs in Abhängigkeit der Information über die Körperregion des Patienten umfassen. Beispielsweise können eine Steuereinheit und/oder eine Recheneinheit der Bildgebungsvorrichtung dazu ausgebildet sein, eine bestimmte Information über eine gewünschte oder ideale Einstellung des Bildgebungsbereichs aus einer Bibliothek oder einer Datenbank in Abhängigkeit der Körperregion des Patienten auszuwählen und bereitzustellen. Es ist vorstellbar, dass die Information über die Körperregion des Patienten einen Namen, eine Bezeichnung und/oder eine Identifikation einer anatomischen Struktur umfasst, welche bei dem Bestimmen der Hilfestellung zur Anpassung des Bildgebungsbereichs mittels der Steuereinheit und/oder Recheneinheit herangezogen werden.

In einem Beispiel umfasst die Information über die Körperregion des Patienten eine Bezeichnung eines Abschnitts einer anatomischen Struktur, insbesondere eines Abschnitts einer Zahnregion oder eines Zahnbogens. Die Hilfestellung zur Anpassung des Bildgebungsbereichs kann entsprechend in Abhängigkeit der Bezeichnung des Abschnitts der anatomischen Struktur ausgewählt und mittels einer Ausgabeeinheit oder einer grafischen Benutzerschnittstelle dem Nutzer der Bildgebungsvorrichtung bereitgestellt werden. Beispielsweise kann die Hilfestellung zur Anpassung des Bildgebungsbereichs aus einer Datenbank einer Speichereinheit, welche eine Mehrzahl von Hilfestellungen zur Anpassung des Bildgebungsbereichs für unterschiedliche Körperregionen umfasst, ausgewählt und abgerufen werden.

Die Hilfestellung zur Anpassung des Bildgebungsbereichs kann dazu ausgebildet sein, einen Nutzer der Bildgebungsvorrichtung über eine gewünschte oder ideale Dimension, Position und/oder Ausrichtung des Bildgebungsbereichs für die Körperregion des Patienten zu informieren. Die Hilfestellung zur Anpassung des Bildgebungsbereichs kann weiterhin dazu ausgebildet sein, den Nutzer der Bildgebungsvorrichtung bei einer Anpassung der Dimension, Position und/oder Ausrichtung des Bildgebungsbereichs relativ zu einer anatomischen Struktur der Körperregion des Patienten zu unterstützen. Es ist vorstellbar, dass die Hilfestellung zur Anpassung des Bildgebungsbereichs eine Richtlinie oder Anweisung darstellt, welche angibt, wie der Bildgebungsbereich für eine Bildgebungsuntersuchung der Körperregion des Patienten oder eines Abschnitts der Körperregion des Patienten parametriert werden sollte. Ein gemäß der Hilfestellung zur Anpassung des Bildgebungsbereichs parametrierter Bildgebungsbereich kann entlang einer diagnostisch relevanten anatomischen Struktur der Körperregion des Patienten ausgerichtet sein und/oder an die diagnostisch relevante anatomische Struktur der Körperregion des Patienten angepasst sein.

In einer bevorzugten Ausführungsform des erfindungsgemäßen Verfahrens umfasst das Bereitstellen der Hilfestellung zur Anpassung des Bildgebungsbereichs eine Überlagerung und/oder Gegenüberstellung der Hilfestellung zur Anpassung des Bildgebungsbereichs mit den Bilddaten der ersten Bildgebungsuntersuchung. Beispielsweise können die Hilfestellung zur Anpassung des Bildgebungsbereichs und die Bilddaten der ersten Bildgebungsuntersuchung einem Nutzer der Bildgebungsvorrichtung mittels einer Ausgabeeinheit oder einer grafischen Benutzerschnittstelle bereitgestellt werden.

Ein Erfassen eines angepassten Bildgebungsbereichs kann ein Erfassen einer Eingabe eines Nutzers der Bildgebungsvorrichtung umfassen. Insbesondere kann die Eingabe des Nutzers mittels einer Eingabeschnittstelle oder Benutzerschnittstelle gemäß einer oben beschriebenen Ausführungsform erfolgen. Die Eingabe des Nutzers kann ein Anpassen eines Parameters oder einer Eigenschaft eines grafischen Objekts, welches den Bildgebungsbereich repräsentiert, umfassen. Insbesondere kann die Eingabe ein Anpassen einer Position, einer Dimension und/oder einer Ausrichtung des grafischen Objekts darstellen. Das grafische Objekt kann ein Fenster oder ein beliebiges Polygon, insbesondere ein Quadrat, ein Rechteck oder ein anderes Vieleck, umfassen. Vorzugsweise ist das grafische Objekt den Bilddaten der ersten Bildgebungsuntersuchung überlagert. Das grafische Objekt kann insbesondere mit der Eingabeoption zur Anpassung des Parameters eines Bildgebungsbereichs übereinstimmen.

Es ist weiterhin vorstellbar, dass die Eingabe des Nutzers eine textbasierte Eingabe umfasst. Die textbasierte Eingabe kann eine oder mehrere Koordinaten, eine oder mehrere Abmessungen des Bildgebungsbereichs, eine Koordinate eines geometrischen Mittelpunkts des Bildgebungsbereichs, einen Rotationswinkel des Bildgebungsbereichs oder dergleichen umfassen.

Vorzugsweise ermöglicht das erfindungsgemäße Verfahren ein Anpassen des Bildgebungsbereichs durch einen Nutzer der Bildgebungsvorrichtung in Abhängigkeit der Hilfestellung zur Anpassung des Bildgebungsbereichs und der Bilddaten der ersten Bildgebungsuntersuchung. Dadurch kann auf vorteilhafte Weise eine Anpassung des Bildgebungsbereichs unter Berücksichtigung sowohl von Vorschriften oder Vorgaben zur Einstellung des Bildgebungsbereichs für bestimmte Körperregionen als auch von individuellen Voraussetzungen einer anatomischen Struktur der Körperregion des Patienten ermöglicht werden.

Das Bestimmen des Bildgebungsprotokolls für eine zweite Bildgebungsuntersuchung kann ein Bestimmen einer oder mehrerer Bildgebungsparameter für die zweite Bildgebungsuntersuchung umfassen. Es ist ebenso vorstellbar, dass das Bestimmen des Bildgebungsprotokolls ein Bestimmen einer oder mehrerer Bildgebungssequenzen, insbesondere einer Abfolge mehrerer Bildgebungssequenzen, umfasst. Das Bildgebungsprotokoll kann einen oder mehrere Bildgebungsparameter, aber auch einen Workflow, der zweiten Bildgebungsuntersuchung oder weiterer Bildgebungsuntersuchungen bestimmen. Die zweite Bildgebungsuntersuchung, aber auch die weiteren Bildgebungsuntersuchungen, können dazu ausgebildet sein, hochaufgelöste Bilddaten der Körperregion des Patienten zu erfassen.

Das Bestimmen des Bildgebungsprotokolls erfolgt erfindungsgemäß in Abhängigkeit des angepassten Bildgebungsbereichs. Vorzugsweise wird in Abhängigkeit des durch den Nutzer angepassten Bildgebungsbereichs automatisch das Bildgebungsprotokoll bestimmt. Beispielsweise können in Abhängigkeit des angepassten Bildgebungsbereichs (z. B. basierend auf der Eingabe des Nutzers) eine oder mehrere Gruppen von Bildgebungsparametern, eine oder mehrere Bildgebungssequenzen und/oder eine Abfolge von Bildgebungssequenzen ermittelt werden. Vorzugsweise erfolgt das Ermitteln des Bildgebungsprotokolls in Abhängigkeit des angepassten Bildgebungsbereichs mittels eines auf einer Steuereinheit und/oder einer Recheneinheit der Bildgebungsvorrichtung implementierten Algorithmus.

Ein hierin erwähnter Algorithmus kann einen Logik-basierten Algorithmus, einen trainierten Algorithmus, einen selbstlernenden Algorithmus, ein künstliches neuronales Netzwerk, einen Machine-Learning Algorithmus, einen Bildverarbeitungsalgorithmus, aber auch einen oder mehrere mathematische Operatoren, umfassen.

Bestimmte Körperregionen von Patienten können sich individuell signifikant unterscheiden. Beispielsweise können eine Größe und/oder ein Geschlecht eines Patienten, aber auch ein Körperumfang eine Lage bestimmter Körperregionen beeinflussen. Es ist jedoch ebenso vorstellbar, dass anatomische Strukturen der Körperregion des Patienten unterschiedliche Formen, Abmessungen oder räumliche Ausrichtungen aufweisen. Weiterhin sind anatomische Strukturen, welche in beiden Körperhälften des Patienten vorliegen, häufig nicht ganz symmetrisch zueinander und können einen Nutzer bei der Bestimmung eines Bildgebungsprotokolls verwirren oder irritieren. Insbesondere anatomische Strukturen, welche sich in mehrere, annähernd ähnliche oder symmetrische, diagnostisch relevanten Bereiche unterteilen lassen, erfordern ein intensives Training von Nutzern der Bildgebungsvorrichtung, um ein Erfassen von Bilddaten der korrekten Körperregion und eine hohe Qualität der erfassten Bilddaten zu gewährleisten.

Ein erfindungsgemäßes Verfahren ermöglicht eine geführte Anleitung eines Nutzers bei der Bestimmung eines Bildgebungsprotokolls zur Erfassung von Bilddaten einer Körperregion eines Patienten mittels einer Bildgebungsvorrichtung. Insbesondere kann das erfindungsgemäße Verfahren den Nutzer dynamisch, also in Abhängigkeit unterschiedlicher diagnostisch relevanter Körperregionen des Patienten und/oder in Abhängigkeit von Bilddaten einer spezifischen Körperregion eines Patienten, bei der Anpassung eines Bildgebungsbereichs unterstützen. Beispielsweise kann der Nutzer durch die Hilfestellung zur Anpassung des Bildgebungsbereichs unmittelbar auf die diagnostisch relevante Körperregion und eine fachübliche Parametrierung des Bildgebungsbereichs hingewiesen werden, ohne den Bildgebungsbereich im Vorfeld festzulegen oder einzuschränken. Dies kann insbesondere bei einer Bildgebungsuntersuchung von Zähnen, welche zwischen Patienten sehr unterschiedlich ausfallen können und aufgrund der Symmetrie zwischen verschiedenen Zahnabschnitten leicht zu Verwechselungen führen können, von Vorteil sein.

Weiterhin kann das erfindungsgemäße Verfahren einem Nutzer einer Bildgebungsvorrichtung ohne spezielle Vorkenntnisse ermöglichen, einen Bildgebungsbereich auch bei Vorliegen komplexer anatomischer Strukturen und/oder Schichtausrichtungen, adäquat zu bestimmen. Dadurch lassen sich Fehler bei dem Bestimmen eines Bildgebungsprotokolls und einem nachfolgenden Erfassen von weiteren Bilddaten der Körperregion vorteilhaft vermeiden. Ferner kann eine hohe Qualität der mittels der Bildgebungsvorrichtung erfassten, weiteren Bilddaten gewährleisten werden.

Mittels des erfindungsgemäßen Verfahrens lassen sich die Bilddaten der Körperregion, die Eingabeoption zur Anpassung des Parameters des Bildgebungsbereichs und die Hilfestellung zur Anpassung des Bildgebungsbereichs gleichzeitig oder in einer vorbestimmten Reihenfolge dem Nutzer der Bildgebungsvorrichtung bereitstellen. Dadurch kann dem Nutzer auf vorteilhafte Weise ein effizienteres und/oder genaueres Anpassen des Bildgebungsbereichs an einen oder mehrere Abschnitte der Körperregion des Patienten ermöglicht werden. Insbesondere kann der Nutzer im Rahmen einer geführten Mensch-Maschine-Interaktion in Abhängigkeit der Bilddaten der Körperregion, der Eingabeoption zur Anpassung des Parameters des Bildgebungsbereichs und der Hilfestellung zur Anpassung des Bildgebungsbereichs mittels einer Benutzerschnittstelle der Bildgebungsvorrichtung bei einem Anpassen des Bildgebungsbereichs an einen oder mehrere Abschnitte der Körperregion des Patienten unterstützt werden.

In einer Ausführungsform umfasst das erfindungsgemäße Verfahren den Schritt:
- Bestimmen eines vorläufigen Bildgebungsbereichs in Abhängigkeit der Bilddaten,
wobei das Bereitstellen der Eingabeoption zur Anpassung des Parameters des Bildgebungsbereichs eine Überlagerung einer Repräsentation des vorläufigen Bildgebungsbereichs mit den Bilddaten der Körperregion des Patienten umfasst.

Das Bestimmen des vorläufigen Bildgebungsbereichs kann insbesondere ein Bestimmen einer Dimension, einer räumlichen Anordnung und/oder einer räumlichen Ausrichtung des Bildgebungsbereichs relativ zu der Körperregion des Patienten umfassen.

Vorzugsweise wird die Repräsentation des vorläufigen Bildgebungsbereichs bei dem Bereitstellen der Bilddaten mit den Bilddaten der Körperregion des Patienten überlagert. Eine Repräsentation des vorläufigen Bildgebungsbereichs kann beispielsweise ein Symbol, ein grafisches Objekt oder eine grafische Darstellung des vorläufigen Bildgebungsbereichs umfassen. Insbesondere kann die Repräsentation des vorläufigen Bildgebungsbereichs ein Fenster oder ein Polygon gemäß einer oben beschriebenen Ausführungsform aufweisen. Das Bereitstellen einer Überlagerung des vorläufigen Bildgebungsbereichs mit den Bilddaten der ersten Bildgebungsuntersuchung kann einen Nutzer der Bildgebungsvorrichtung über eine bevorzugte oder konventionelle Dimension, Orientierung und/oder räumliche Lage des vorläufigen Bildgebungsbereichs in Relation zu der Körperregion des Patienten informieren.

In einer bevorzugten Ausführungsform wird die Repräsentation des vorläufigen Bildgebungsbereichs den Bilddaten der Körperregion des Patienten überlagert und zusammen mit der Eingabeoption zur Anpassung des Parameters des Bildgebungsbereichs bereitgestellt. Dadurch lässt sich ein Startpunkt für die Anpassung des Bildgebungsbereichs durch den Nutzer bereitstellen und eine Effizienz einer Anpassung des Bildgebungsbereichs auf vorteilhafte Weise erhöhen.

Es ist vorstellbar, dass das Bestimmen des vorläufigen Bildgebungsbereichs in Abhängigkeit der Bilddaten mittels eines Algorithmus, insbesondere eines Bildverarbeitungsalgorithmus, erfolgt. Beispielsweise kann der Algorithmus dazu ausgebildet sein, die Bilddaten oder Localizer-Bilddaten zu verarbeiten und eine Dimension, eine räumliche Anordnung und/oder eine Ausrichtung des vorläufigen Bildgebungsbereichs relativ zu der Körperregion des Patienten zu ermitteln. Es ist weiterhin vorstellbar, dass das Bestimmen des vorläufigen Bildgebungsbereichs in Abhängigkeit einer Vorgabe oder Richtlinie zur Parametrierung eines Bildgebungsbereichs für eine spezifische Körperregion, einer Referenz-Bildgebungsuntersuchung der gleichen Körperregion eines anderen Patienten und/oder einer geometrischen Analyse der Körperregion des Patienten erfolgt.

Das Bereitstellen einer Hilfestellung zur Anpassung des Bildgebungsbereichs und einer den Bilddaten überlagerten Repräsentation eines vorläufigen Bildgebungsbereichs kann insbesondere unerfahrenen Nutzern ein Anpassen des Bildgebungsbereichs bei komplex oder untypisch aufgebauten anatomischen Strukturen, aber auch bei schwierigen Schichtausrichtungen, ermöglichen oder erleichtern.

Weiterhin ermöglicht das Bereitstellen der Hilfestellung zur Anpassung des Bildgebungsbereichs und des vorläufigen Bildgebungsbereichs dem Nutzer eine dynamische Bewertung etwaiger Differenzen zwischen der Hilfestellung zur Anpassung des Bildgebungsbereichs und dem vorläufigen Bildgebungsbereich, welche z. B. infolge komplex oder untypisch gestalteter anatomischer Strukturen auftreten können. Dadurch kann der Nutzer vorteilhaft auf Probleme bei der Anpassung des Bildgebungsbereichs hingewiesen und ein Risiko einer fehlerhaften Bestimmung des Bildgebungsprotokolls reduziert werden.

In einer weiteren Ausführungsform des erfindungsgemäßen Verfahrens umfasst das Erfassen des angepassten Bildgebungsbereichs ein Erfassen einer Korrektur des vorläufigen Bildgebungsbereichs.

Vorzugsweise umfasst das Erfassen des angepassten Bildgebungsbereichs ein Erfassen einer Korrektur einer vorläufigen Dimension, einer vorläufigen räumlichen Anordnung und/oder einer vorläufigen Ausrichtung des Bildgebungsbereichs relativ zu der Körperregion des Patienten.

Insbesondere kann das Bereitstellen der Eingabeoption zur Anpassung des Parameters des Bildgebungsbereichs eine Aufforderung des Nutzers der Bildgebungsvorrichtung zu einer Anpassung oder Korrektur des vorläufigen Bildgebungsbereichs umfassen. Die Eingabeoption zur Anpassung des Parameters des Bildgebungsbereichs kann dem Nutzer gemäß einer oben beschriebenen Ausführungsform mittels einer Ausgabeeinheit und/oder einer grafischen Benutzerschnittstelle bereitgestellt werden.

Diagnostisch relevante Körperregionen mit geringen Abmessungen, wie z. B. eine Entzündung einer Pulpa oder einer Zahnwurzel, müssen typischerweise bei der Bestimmung eines Bildgebungsprotokolls berücksichtigt werden und lassen sich nicht ohne Weiteres mittels konventioneller Verfahren zur Bestimmung des Bildgebungsbereichs abdecken. Bei anatomischen Strukturen, deren Abmessungen und/oder Orientierung von einer typischen Norm oder Standardverteilung abweichen, wird der Prozess der Bestimmung des Bildgebungsbereichs zusätzlich erschwert.

Ein erfindungsgemäßes Verfahren ermöglicht eine Korrektur eines vorläufigen Bildgebungsbereichs unter Zuhilfenahme einer bereitgestellten Hilfestellung zur Anpassung des Bildgebungsbereichs. Insbesondere ermöglicht ein direkter Vergleich der Bilddaten der Körperregion des Patienten mit dem vorläufigen Bildgebungsbereich und der Hilfestellung zur Anpassung des Bildgebungsbereichs dem Nutzer auf vorteilhafte Weise eine dynamische Bewertung erforderlicher Maßnahmen zur individuellen Korrektur oder Anpassung des Bildgebungsbereichs für eine spezifische anatomische Struktur eines Patienten. Dadurch lässt sich eine korrekte Zuordnung des Bildgebungsbereichs auch bei anatomischen Strukturen, welche von einer Norm abweichen, sicherstellen.

In einer bevorzugten Ausführungsform weist das erfindungsgemäße Verfahren den weiteren Schritt auf:
- Durchführen der zweiten Bildgebungsuntersuchung zum Erfassen von weiteren Bilddaten der Körperregion des Patienten in Abhängigkeit des Bildgebungsprotokolls.

Die weiteren Bilddaten können sich von den Bilddaten der ersten Bildgebungsuntersuchung insbesondere durch eine höhere Qualität, eine verbesserte Zentrierung auf eine diagnostisch relevante anatomische Struktur und/oder eine erhöhte räumliche Auflösung unterscheiden.

Es ist vorstellbar, dass die erste Bildgebungsuntersuchung eine Localizer-Messung umfasst, während die zweite Bildgebungsuntersuchung eine auf die Körperregion oder einen Abschnitt der Körperregion des Patienten fokussierte, hochauflösende Bildgebungsuntersuchung umfasst. Die Localizer-Messung kann insbesondere die Funktion erfüllen, dem Nutzer durch Bereitstellen der Bilddaten mit der spezifischen Gestalt der Körperregion des Patienten eine Anpassung eines Parameters des Bildgebungsprotokolls für die zweite Bildgebungsuntersuchung zu ermöglichen.

Die erste Bildgebungsuntersuchung und die zweite Bildgebungsuntersuchung werden vorzugsweise mittels derselben Bildgebungsvorrichtung durchgeführt. Es ist jedoch ebenso vorstellbar, dass die erste Bildgebungsuntersuchung und die zweite Bildgebungsuntersuchung mittels unterschiedlicher Bildgebungsvorrichtungen (z. B. unterschiedlicher Magnetresonanzgeräte) oder unterschiedlicher Bildgebungsmodalitäten (z. B. einem Röntgengerät und einem Magnetresonanzgerät) ausgeführt werden. Hierfür kann der Patient beispielsweise mittels einer stereotaktischen Patientenaufnahmevorrichtung in einer reproduzieren relativen Position zu der ersten Bildgebungsvorrichtung und der zweiten Bildgebungsvorrichtung positioniert werden. Ferner kann können Marker verwendet werden, um ein Anpassen des Bildgebungsbereichs für die Bestimmung des Bildgebungsprotokolls für die zweite Bildgebungsuntersuchung mittels der zweiten Bildgebungsvorrichtung zu ermöglichen. Die erste Bildgebungsuntersuchung und die zweite Bildgebungsuntersuchung können somit zu unterschiedlichen Zeitpunkten und/oder an unterschiedlichen Orten durchgeführt werden.

Ein Durchführen einer zweiten Bildgebungsuntersuchung in Abhängigkeit eines auf Basis einer ersten Bildgebungsuntersuchung bestimmten Bildgebungsprotokolls ermöglicht ein Anpassen der zweiten Bildgebungsuntersuchung an individuelle Voraussetzungen einer Körperregion eines Patienten. Insbesondere erlaubt das erfindungsgemäße Verfahren eine reproduzierbare Aufnahme von weiteren Bilddaten der Körperregion mit hoher Qualität, unabhängig von einer Erfahrung des Nutzers der Bildgebungsvorrichtung.

In einer Ausführungsform des erfindungsgemäßen Verfahrens umfasst das Bereitstellen der Hilfestellung zur Anpassung des Bildgebungsbereichs ein Ausgeben einer textbasierten Anleitung und/oder einer grafischen Anleitung.

Eine textbasierte Anleitung kann eine Beschreibung oder eine Anweisung umfassen, welche eine Information über einen gewünschten oder idealen Parameter des Bildgebungsbereichs für ein bestimmtes Bildgebungsprotokoll, eine bestimmte Körperregion und/oder einen bestimmten Abschnitt einer Körperregion enthält. Insbesondere kann die textbasierte Anleitung einen Vorschlag oder eine Richtlinie zur Anpassung eines Parameters des Bildgebungsbereichs und/oder eines Vorgehens zur Anpassung eines Parameters des Bildgebungsbereichs in Abhängigkeit einer zu untersuchenden Körperregion und/oder einem Abschnitt einer zu untersuchenden Körperregion enthalten. Beispielsweise kann die textbasierte Anleitung eine Beschreibung eines gewünschten oder idealen Parameters des Bildgebungsbereichs für einen bestimmten Abschnitt einer anatomischen Struktur des Patienten, insbesondere eines Abschnitts eines Zahnbogens oder Abschnitte beider Zahnbögen, umfassen.

Ein Parameter des Bildgebungsbereichs kann eine Dimension, eine räumliche Position und/oder eine Ausrichtung des Bildgebungsbereichs relativ zu der Körperregion des Patienten definieren. Ein Parameter des Bildgebungsbereichs kann insbesondere einen Bildgebungsparameter eines Bildgebungsprotokolls darstellen.

Eine grafische Anleitung kann eine piktographische oder bildhafte Information über einen gewünschten oder idealen Parameter des Bildgebungsbereichs für ein bestimmtes Bildgebungsprotokoll, eine bestimmte Körperregion und/oder einen bestimmten Abschnitt einer Körperregion umfassen. Vorzugsweise umfasst die grafische Anleitung eine gewünschte oder ideale Dimension, eine gewünschte oder ideale räumliche Position und/oder eine gewünschte oder ideale Ausrichtung des Bildgebungsbereichs relativ zu der Körperregion des Patienten.

Sowohl die textbasierte Anleitung als auch die grafische Anleitung können einen Nutzer der Bildgebungsvorrichtung auf einen gewünschten oder idealen Parameter des Bildgebungsbereichs in Abhängigkeit der Körperregion des Patienten hinweisen. Der Nutzer kann jedoch in Abhängigkeit der individuellen anatomischen Voraussetzungen der Körperregion des Patienten entscheiden (z. B. in Abhängigkeit der Bilddaten der ersten Bildgebungsuntersuchung), bei der Anpassung des Bildgebungsbereichs von dem gewünschten oder idealen Parameter des Bildgebungsbereichs abzuweichen.

Durch das Bereitstellen einer erfindungsgemäßen textbasierten und/oder einer grafischen Anleitung können auch unerfahrene Nutzer über eine fachübliche Parametrierung des Bildgebungsbereichs in Abhängigkeit der Körperregion informiert werden. Dadurch lassen sich Kosten für ein Training des Personals, aber auch Fehler bei der Bestimmung des Bildgebungsprotokolls, auf vorteilhafte Weise reduzieren.

In einer bevorzugten Ausführungsform des erfindungsgemäßen Verfahrens umfasst das Bereitstellen der Hilfestellung zur Anpassung des Bildgebungsbereichs das Ausgeben einer grafischen Anleitung, wobei die grafische Anleitung eine Repräsentation der Körperregion und des Bildgebungsbereichs umfasst.

Die grafische Anleitung kann insbesondere eine detaillierte oder abstrakte grafische Darstellung des Bildgebungsbereichs in einer gewünschten Position und/oder Ausrichtung zu der Körperregion des Patienten umfassen. Vorzugsweise umfasst die grafische Anleitung ferner eine Repräsentation der Körperregion des Patienten in einer korrekten räumlichen Anordnung zu der abstrakten grafischen Darstellung des Bildgebungsbereichs. Weiterhin kann die grafische Anleitung ein Symbol und/oder ein Zeichen umfassen, welches den Nutzer über eine Änderung eines Parameters des Bildgebungsbereichs zur Erreichung einer gewünschten oder idealen relativen Anordnung des Bildgebungsbereichs zu der Körperregion des Patienten informiert. Beispielsweise kann die grafische Anleitung einen Pfeil und/oder eine Linie enthalten, welche den Nutzer auf eine Änderung einer Orientierung, einer Größe und/oder einer Position des Bildgebungsbereichs hinweisen.

Eine grafische Anleitung erlaubt eine Anpassung des Bildgebungsbereichs durch eine direkte visuelle Gegenüberstellung einer Anordnung und/oder einer Gestalt der Repräsentation des Bildgebungsbereichs mit den Bilddaten der ersten Bildgebungsuntersuchung und/oder einem vorläufigen Bildgebungsbereich gemäß einer oben beschriebenen Ausführungsform. Dadurch lässt sich ein Prozess einer Anpassung des Bildgebungsparameters vorteilhaft vereinfachen oder beschleunigen. Ferner lässt sich eine Verwendung von Fachwörtern, welche bei einer Verwendung einer textbasierten Anleitung erforderlich werden können, vorteilhaft vermeiden. Dadurch kann die Anpassung des Bildgebungsbereichs auf vorteilhafte Weise auch von unerfahrenen Nutzern vorgenommen werden.

Ein weiterer Vorteil einer grafischen Anleitung mit einer Repräsentation der Körperregion des Patienten besteht in der Möglichkeit, den idealen oder gewünschten Bildgebungsbereich relativ zu der Körperregion des Patienten den Bilddaten der Körperregion des Patienten, aber auch dem vorläufigen Bildgebungsbereich, gegenüberzustellen. Die grafische Anleitung kann auf vorteilhafte Weise Probleme bei der Anpassung des Bildgebungsbereichs an eine anatomische Struktur des Patienten aufzeigen, welche dem Nutzer bei einer textbasierten oder einer Parameter-basierten Anpassung des Bildgebungsbereichs verborgen blieben.

In einer bevorzugten Ausführungsform des erfindungsgemäßen Verfahrens umfasst die grafische Anleitung zumindest zwei Schnittansichten der Körperregion des Patienten. Die zumindest zwei Schnittansichten der Körperregion des Patienten sind entlang unterschiedlicher, vorzugsweise orthogonal zueinander ausgerichteter, Bezugsebenen ausgerichtet.

Beispielsweise ist eine Bezugsebene einer ersten Schnittansicht der zumindest zwei Schnittansichten der Körperregion parallel zu einer Frontalebene des Patienten angeordnet. Eine Bezugsebene einer zweiten Schnittansicht der zumindest zwei Schnittansichten der Körperregion kann parallel zu einer Sagittalebene des Patienten angeordnet sein. Es ist ebenso vorstellbar, dass die grafische Anleitung eine dritte Schnittansicht der Körperregion des Patienten umfasst. Eine Bezugsebene der dritten Schnittansicht der Körperregion kann parallel zu einer Transversalebene des Patienten ausgerichtet sein. Selbstverständlich kann die grafische Anleitung auch nur eine Schnittansicht oder nur zwei Schnittansichten der Körperregion umfassen, deren Bezugsebenen jeweils parallel zu der Sagittalebene, der Frontalebene oder der Transversalebene des Patienten angeordnet sind.

Vorzugsweise sind die Bezugsebenen der zumindest zwei Schnittansichten der Körperregion im Wesentlichen orthogonal zueinander ausgerichtet. Dies kann bedeuten, dass eine Ausrichtung der Bezugsebenen der ersten Schnittansicht und der zweiten Schnittansicht um wenige Grad, beispielsweise weniger als 10°, weniger als 8°, weniger als 6° oder weniger als 4°, von einer rechtwinkligen Ausrichtung abweichen.

Es ist weiterhin vorstellbar, dass ein durch die Bezugsebenen der zumindest zwei Schnittansichten der Körperregion definiertes Koordinatensystem um einen Winkel gegenüber einem Koordinatensystem, welches durch die Sagittalebene, die Frontalebene und die Transversalebene des Patienten definiert ist, geneigt ist.

Ein Bereitstellen einer erfindungsgemäßen grafischen Anleitung erlaubt eine Veranschaulichung einer gewünschten oder idealen Anordnung des Bildgebungsbereichs in zumindest zwei Bezugsebenen. Dadurch wird es einem Nutzer der Bildgebungsvorrichtung auf vorteilhafte Weise ermöglicht, eine Differenz einer aktuellen Anordnung des Bildgebungsbereichs und/oder eines vorläufigen Bildgebungsbereichs zu der gewünschten oder idealen Anordnung des Bildgebungsbereichs hinsichtlich der Bezugsebenen der zumindest zwei Schnittansichten unter Berücksichtigung einer individuellen Gestalt der Körperregion des Patienten zu ermitteln. Insbesondere kann das Bereitstellen mehrerer Schnittansichten der Körperregion einem Nutzer der Bildgebungsvorrichtung ein genaueres Anpassen des Bildgebungsbereichs an individuelle Formen von anatomischen Strukturen, wie z. B. einem Zahnbogen des Patienten, ermöglichen.

In einer weiteren Ausführungsform des erfindungsgemäßen Verfahrens umfasst das Bereitstellen der Bilddaten und der Eingabeoption zur Anpassung des Parameters des Bildgebungsbereichs ein Ausgeben von zumindest zwei auf den Bilddaten basierenden Schnittansichten der Körperregion des Patienten, wobei je eine Bezugsebene der zumindest zwei auf den Bilddaten basierenden Schnittansichten der Körperregion des Patienten parallel zu je einer Bezugsebene der zumindest zwei Schnittansichten der Körperregion des Patienten der grafischen Anleitung ausgerichtet ist.

Beispielsweise ist eine Bezugsebene einer ersten auf den Bilddaten basierenden Schnittansicht der Körperregion im Wesentlichen parallel zu der Bezugsebene der ersten Schnittansicht der Körperregion der grafischen Anleitung ausgerichtet. Gleichermaßen kann eine Bezugsebene einer zweiten auf den Bilddaten basierenden Schnittansicht der Körperregion im Wesentlichen parallel zu der Bezugsebene der zweiten Schnittansicht der Körperregion der grafischen Anleitung ausgerichtet sein.

In einer bevorzugten Ausführungsform umfasst das Bereitstellen der Bilddaten und der Eingabeoption zur Anpassung des Parameters des Bildgebungsbereichs ein Ausgeben einer dritten auf den Bilddaten basierenden Schnittansicht der Körperregion, wobei eine Bezugsebene der dritten auf den Bilddaten basierenden Schnittansicht der Körperregion parallel zu einer Bezugsebene einer dritten Schnittansicht der Körperregion der grafischen Anleitung ausgerichtet ist.

In einer besonders bevorzugten Ausführungsform korrespondiert die Bezugsebene der ersten auf den Bilddaten basierenden Schnittansicht der Körperregion mit der Bezugsebene der ersten Schnittansicht Körperregion der grafischen Anleitung. Gleichermaßen können die Bezugsebenen der zweiten und/oder dritten auf den Bilddaten basierenden Schnittansichten der Körperregion mit den jeweiligen Bezugsebenen der zweiten und/oder dritten Schnittansicht der Körperregion der grafischen Anleitung korrespondieren.

Eine auf den Bilddaten basierende Schnittansicht der Körperregion kann ein zweidimensionales Bild, beispielsweise ein Magnetresonanzbild, ein Röntgenbild oder ein Computertomografie-Bild, der Körperregion des Patienten darstellen. Es ist vorstellbar, dass die auf den Bilddaten basierende Schnittansicht der Körperregion ein transversales Schnittbild, ein frontales Schnittbild und/oder ein sagittales Schnittbild der Körperregion umfasst. Die auf den Bilddaten basierende Schnittansicht der Körperregion kann insbesondere ein transversales Schnittbild, ein frontales Schnittbild und/oder ein sagittales Schnittbild einer Localizer-Messung der Körperregion des Patienten umfassen.

Eine grafische Anleitung mit einer Repräsentation der Körperregion des Patienten kann signifikant von einer individuellen Gestalt und/oder Anordnung der Körperregion in einem Patienten abweichen. Durch ein Bereitstellen einer grafischen Anleitung mit Schnittansichten der Körperregion und dazu korrespondierenden, auf den Bilddaten basierenden Schnittansichten der Körperregion, lässt sich eine Anordnung und/oder Ausrichtung des Bildgebungsbereichs in mehreren Raumrichtungen an ein gewünschtes oder ideales Ergebnis annähern. Weiterhin können bei der Anpassung des Bildgebungsbereichs gleichzeitig individuelle Voraussetzungen einer Gestalt und/oder Ausrichtung der Körperregion und/oder eines Abschnitts der Körperregion des Patienten berücksichtigt werden. Dadurch lässt sich eine Effizienz der Anpassung des Bildgebungsbereichs auf vorteilhafte Weise erhöhen und/oder ein Zeitaufwand eines Nutzers der Bildgebungsvorrichtung für ein Vorbereiten oder Parametrieren der zweiten Bildgebungsuntersuchung reduzieren.

In einer bevorzugten Ausführungsform des erfindungsgemäßen Verfahrens umfasst das Bereitstellen der Bilddaten und der Eingabeoption zur Anpassung des Parameters des Bildgebungsbereichs ein Gegenüberstellen der Bilddaten mit der grafischen Anleitung.

Vorzugsweise umfasst die grafische Anleitung eine Schnittansicht der Körperregion des Patienten gemäß einer oben beschriebenen Ausführungsform. Gleichermaßen können die Bilddaten eine Schnittansicht der Körperregion des Patienten umfassen. Die Bezugsebenen der Schnittansicht der Körperregion der grafischen Anleitung und der auf den Bilddaten basierenden Schnittansicht der Körperregion können gemäß einer oben beschriebenen Ausführungsform parallel zueinander ausgerichtet sein oder miteinander korrespondieren.

Es ist vorstellbar, dass die Bilddaten zusammen mit der grafischen Anleitung dem Nutzer der Bildgebungsvorrichtung mittels einer Ausgabeeinheit oder einer grafischen Benutzerschnittstelle bereitgestellt werden. Die Bilddaten und die grafische Anleitung können dabei nebeneinander angeordnet oder zumindest teilweise überlappend dargestellt werden. Vorzugsweise werden die Bilddaten und die grafische Anleitung derart mittels der Ausgabeeinheit oder der grafischen Benutzerschnittstelle bereitgestellt, dass die Bilddaten und die grafische Anleitung gemeinsam auf einem dem Nutzer zu einem bestimmten Zeitpunkt sichtbaren Anzeigebereich dargestellt werden.

Eine erfindungsgemäße Gegenüberstellung der Bilddaten und der grafischen Anleitung ermöglicht eine visuelle Anpassung des Bildgebungsbereichs an eine individuelle anatomische Struktur der Körperregion des Patienten unter gleichzeitiger Berücksichtigung einer gewünschten oder idealen Anordnung und/oder Ausrichtung des Bildgebungsbereichs an einen durch die grafische Anleitung veranschaulichten Standardfall. Dadurch lassen sich Verwechselungen bei der Auswahl von Abschnitten der Körperregion, aber auch Fehler bei der Anpassung des Bildgebungsbereichs, auf vorteilhafte Weise reduzieren oder vermeiden.

Eine gewünschte oder ideale Anordnung und/oder Ausrichtung des Bildgebungsbereichs bezüglich einer spezifischen Körperregion kann durch eine Vorgabe oder eine Richtlinie einer Fachperson, einer Einrichtung, einer Institution und/oder eines Nutzergremiums definiert sein.

In einer weiteren Ausführungsform des erfindungsgemäßen Verfahrens umfasst die Körperregion einen Anatomiebereich einer ersten Körperhälfte des Patienten, welcher im Wesentlichen symmetrisch zu einem Anatomiebereich in einer der ersten Körperhälfte gegenüberliegenden zweiten Körperhälfte des Patienten aufgebaut ist.

Vorzugsweise ist die erste Körperhälfte des Patienten im Wesentlichen symmetrisch zu der zweiten Körperhälfte des Patienten ausgestaltet. Es ist vorstellbar, dass sich die erste Körperhälfte und die zweite Körperhälfte des Patienten an einer Medianebene des Patienten symmetrisch gegenüberstehen.

Beispielsweise kann die Körperregion des Patienten ein Teil einer Extremität, wie z. B. einen Arm, eine Hand, einen Fuß, ein Knie, eine Schulter oder dergleichen, umfassen. Weiterhin kann die Körperregion ein Gelenk, wie z. B. ein Kniegelenk, ein Schultergelenk, ein Hüftgelenk, ein Handgelenk, aber auch Teil eines Organs, wie z. B. eine Gehirnhälfte, eine Lungenhälfte oder dergleichen, umfassen.

In einer bevorzugten Ausführungsform des erfindungsgemäßen Verfahrens umfasst die Körperregion des Patienten eine Kieferregion und/oder eine Zahnregion. Beispielsweise umfassen die Kieferregion und/oder die Zahnregion ein Gebiss, einen Abschnitt eines Kieferknochens, einen Abschnitt eines Gebisses, einen oder mehrere Zahnbögen, einen Abschnitt eines Zahnbogens, eine Gingiva, einen Abschnitt einer Gingiva und/oder einen oder mehrere Zähne des Patienten.

Eine Zahnregion eines Patienten weist zwei im Wesentlichen gleichförmige Zahnbögen auf, welche wiederum Abschnitte auf der linken Körperhälfte des Patienten und der rechten Körperhälfte des Patienten aufweisen. Zudem sind Reihen von Frontzähnen der Zahnbögen unter einem Winkel zu Reihen von Backenzähnen der Zahnbögen angeordnet, weshalb zahlreiche Abschnitte der Zahnregion bei der Bildgebungsuntersuchung der Zahnregion unterschieden werden müssen. Die Bilddaten solcher Abschnitte der Zahnregion können optisch sehr ähnlich sein, wodurch ein Risiko einer Verwechslung einer rechten Seite mit einer linken Seite eines Zahnbogens oder eines Abschnitts eines unteren Zahnbogens mit einem Abschnitt eines oberen Zahnbogens erhöht wird.

Durch das Bereitstellen der Hilfestellung zur Anpassung des Bildgebungsbereichs in Abhängigkeit der Information über die Körperregion kann ein direkter Bezug zu einem für die weitere Bildgebungsuntersuchung relevanten Abschnitt der Körperregion hergestellt werden. Dadurch lässt sich ein Risiko eines Anpassens des Bildgebungsbereichs für einen falschen Abschnitt der Körperregion durch einen Nutzer der Bildgebungsvorrichtung auch bei komplexen anatomischen Strukturen mit unterschiedlichen Abschnitten auf vorteilhafte Weise reduzieren oder vermeiden.

In einer bevorzugten Ausführungsform des erfindungsgemäßen Verfahrens ist eine Bezugsebene einer ersten Schnittansicht der zumindest zwei Schnittansichten der Körperregion des Patienten parallel zu einer Okklusionsebene des Patienten entlang eines Abschnitts eines Zahnbogens ausgerichtet. Eine Bezugsebene einer zweiten Schnittansicht der zumindest zwei Schnittansichten der Körperregion des Patienten ist orthogonal zu der ersten Schnittansicht der Körperregion des Patienten ausgerichtet.

Wie oben beschrieben kann ein durch die Bezugsebenen der zumindest zwei Schnittansichten der Körperregion definiertes Koordinatensystem um einen Winkel gegenüber einem Koordinatensystem, welches durch die Sagittalebene, die Frontalebene und die Transversalebene des Patienten definiert ist, geneigt sein. Der Winkel der Neigung kann insbesondere einem Winkel zwischen einer durch die Okklusionsebene des Patienten definierte Ebene und einer Transversalebene des Patienten entsprechen.

Das Bereitstellen einer parallel zu einer Okklusionsebene des Patienten entlang eines Abschnitts eines Zahnbogens ausgerichteten Schnittansicht der Körperregion ermöglicht eine Berücksichtigung einer Ausrichtung des Bildgebungsbereichs relativ zu einer Krümmung oder Bahnkurve eines Zahnbogens, welche sich bei unterschiedlichen Patienten, insbesondere Patienten unterschiedlichen Alters, unterscheiden können. Dadurch kann ein Anpassen des Bildgebungsbereichs entlang einer dreidimensionalen Erstreckung eines diagnostisch relevanten Abschnitts der Körperregion des Patienten durch einen Nutzer auf vorteilhafte Weise vereinfacht und/oder ein Risiko eines Auftretens von Fehlern bei der Anpassung des Bildgebungsbereichs reduziert werden.

Die erfindungsgemäße Bildgebungsvorrichtung ist dazu ausgebildet, Bilddaten einer Körperregion eines Patienten zu erfassen. Die Bildgebungsvorrichtung kann gemäß einer oben beschriebenen Ausführungsform ausgestaltet sein.

In einer bevorzugten Ausführungsform ist die Bildgebungsvorrichtung als ein Magnetresonanzgerät ausgestaltet. Das Magnetresonanzgerät kann dazu ausgebildet sein, Magnetresonanzdaten, insbesondere Magnetresonanzbilder, eines in einem Patientenaufnahmebereich des Magnetresonanzgeräts positionierten Patienten zu erfassen. Die Magnetresonanzdaten oder Magnetresonanzbilddaten können Bilddaten, insbesondere Localizer-Bilddaten, gemäß einer oben beschriebenen Ausführungsform umfassen.

Das Magnetresonanzgerät ist dazu ausgebildet, eine erste Bildgebungsuntersuchungen, aber auch eine zweite Bildgebungsuntersuchung, der Körperregion des Patienten gemäß einer oben beschriebenen Ausführungsform des erfindungsgemäßen Verfahrens durchzuführen. Durch die Verwendung eines Magnetresonanzgeräts kann eine Belastung des Patienten mit ionisierender Strahlung im Vergleich zu Röntgengeräten oder Computertomografie-Geräten auf vorteilhafte Weise vermieden werden.

Die Bildgebungsvorrichtung umfasst eine Steuereinheit, eine Ausgabeeinheit und eine Benutzerschnittstelle. Erfindungsgemäß ist die Steuereinheit dazu ausgebildet, ein Verfahren nach einem der vorhergehenden Ansprüche zu koordinieren und mittels der Bildgebungsvorrichtung auszuführen. Die Steuereinheit kann in die Bildgebungsvorrichtung integriert sein oder als eine eigenständige Komponente ausgestaltet sein.

Die Ausgabeeinheit ist dazu ausgebildet, einem Nutzer der Bildgebungsvorrichtung die Hilfestellung zur Anpassung des Bildgebungsbereichs bereitzustellen. Die Benutzerschnittstelle ist dazu ausgebildet, dem Nutzer ein Anpassen des Bildgebungsbereichs in Abhängigkeit der Hilfestellung zur Anpassung des Bildgebungsbereichs zu ermöglichen.

Die Ausgabeeinheit und die Benutzerschnittstelle können nach einer oben beschriebenen Ausführungsform ausgestaltet sein. Es ist vorstellbar, dass die Ausgabeeinheit und die Benutzerschnittstelle in eine Komponente integriert sind. Die Ausgabeeinheit und die Benutzerschnittstelle können aber auch als eigenständige Komponenten ausgestaltet sein. In einer bevorzugten Ausführungsform bilden die Ausgabeeinheit und die Benutzerschnittstelle eine grafische Benutzerschnittstelle, welche dazu ausgebildet ist, dem Nutzer der Bildgebungsvorrichtung die Hilfestellung zur Anpassung des Bildgebungsbereichs bereitzustellen und ein Anpassen des Bildgebungsbereichs zu ermöglichen.

Vorzugsweise weist die Steuereinheit eine Signalverbindung mit der Benutzerschnittstelle und/oder der Ausgabeeinheit der Bildgebungsvorrichtung auf. Insbesondere kann die Steuereinheit dazu ausgebildet sein, die Ausgabeeinheit anzusteuern, die Hilfestellung zur Anpassung des Bildgebungsbereichs an den Nutzern der Bildgebungsvorrichtung auszugeben. Die Steuereinheit kann weiterhin dazu ausgebildet sein, durch den Nutzer vorgenommene Anpassungen des Bildgebungsbereichs mittels der Benutzerschnittstelle zu erfassen.

Die erfindungsgemäße Bildgebungsvorrichtung teilt die Vorteile eines erfindungsgemäßen Verfahrens nach einer oben beschriebenen Ausführungsform.

Die Komponenten der erfindungsgemäßen Bildgebungsvorrichtung können vorteilhaft aufeinander abgestimmt sein, sodass eine zeiteffiziente und robuste Durchführung eines erfindungsgemäßen Verfahrens ermöglicht wird. Insbesondere kann die erfindungsgemäße Bildgebungsvorrichtung dazu ausgebildet sein, einen Ablauf einzelner Verfahrensschritte autark zu koordinieren und durchzuführen. Dem Nutzer der Bildgebungsvorrichtung kann somit ein Anpassen des Bildgebungsbereichs für eine weitere Bildgebungsuntersuchung der Körperregion des Patienten ermöglicht werden, ohne dass eine besondere Fachkenntnis des Nutzers oder ein aufwändiges Training erforderlich ist.

Das erfindungsgemäße Computerprogrammprodukt ist direkt in eine Speichereinheit einer Recheneinheit einer erfindungsgemäßen Bildgebungsvorrichtung ladbar. Das Computerprogrammprodukt weist Programmcode-Mittel auf, um ein erfindungsgemäßes Verfahren nach einer oben beschriebenen Ausführungsform durchzuführen, wenn das Computerprogrammprodukt in der Recheneinheit der Bildgebungsvorrichtung ausgeführt wird.

Durch das erfindungsgemäße Computerprogrammprodukt kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Vorzugsweise weist die Recheneinheit erforderliche Voraussetzungen, wie beispielsweise einen Arbeitsspeicher, eine Grafikkarte oder eine Logikeinheit, auf, sodass die jeweiligen Verfahrensschritte effizient ausgeführt werden können. Das Computerprogrammprodukt ist beispielsweise auf einem computerlesbaren Medium gespeichert oder auf einem Netzwerk, einem Server oder einer Cloud hinterlegt, von wo es in einen Prozessor der Recheneinheit geladen werden kann. Die Recheneinheit kann dabei als eine eigenständige Systemkomponente oder als ein Teil der Bildgebungsvorrichtung ausgebildet sein. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit der Bildgebungsvorrichtung ein erfindungsgemäßes Verfahren durchführen. Beispiele für elektronisch lesbare Datenträger sind eine DVD, ein Magnetband, ein USB-Stick oder beliebige andere Datenspeicher, auf welchen elektronisch lesbare Steuerinformationen, insbesondere Software, gespeichert ist. Wenn diese Steuerinformationen von dem Datenträger gelesen und an eine Steuereinheit und/oder die Recheneinheit der Bildgebungsvorrichtung übertragen werden, können alle erfindungsgemäßen Ausführungsformen des beschriebenen, erfindungsgemäßen Verfahrens durchgeführt werden.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine schematische Repräsentation einer Ausführungsform einer erfindungsgemäßen Bildgebungsvorrichtung,
- Fig. 2: ein Ablaufdiagramm einer Ausführungsform eines erfindungsgemäßen Verfahrens,
- Fig. 3: eine schematische Repräsentation einer Hilfestellung zur Anpassung des Bildgebungsbereichs einer Ausführungsform eines erfindungsgemäßen Verfahrens,
- Fig. 4: eine schematische Repräsentation einer Hilfestellung zur Anpassung des Bildgebungsbereichs einer Ausführungsform eines erfindungsgemäßen Verfahrens,
- Fig. 5: eine schematische Repräsentation einer Hilfestellung zur Anpassung des Bildgebungsbereichs einer Ausführungsform eines erfindungsgemäßen Verfahrens,
- Fig. 6: eine schematische Repräsentation einer Hilfestellung zur Anpassung des Bildgebungsbereichs einer Ausführungsform eines erfindungsgemäßen Verfahrens,
- Fig. 7: eine schematische Repräsentation einer Hilfestellung zur Anpassung des Bildgebungsbereichs einer Ausführungsform eines erfindungsgemäßen Verfahrens.

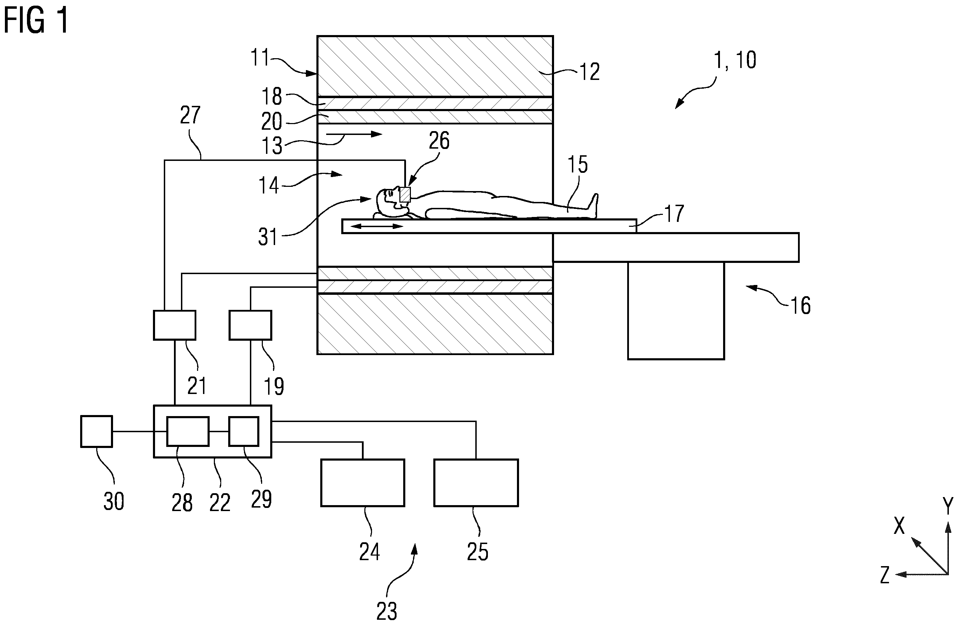

In Fig. 1 ist eine Ausführungsform einer erfindungsgemäßen Bildgebungsvorrichtung 1 dargestellt. Die Bildgebungsvorrichtung 1 ist vorliegend als ein Magnetresonanzgerät 10 ausgestaltet. Das Magnetresonanzgerät 10 umfasst eine Magneteinheit 11, welche z. B. einen Permanentmagneten, einen Elektromagneten oder einen supraleitenden Hauptmagneten 12 zur Erzeugung eines starken und insbesondere homogenen Hauptmagnetfelds 13 (B0-Magnetfeld) aufweist. Zudem umfasst das Magnetresonanzgerät 10 einen Patientenaufnahmebereich 14 zu einer Aufnahme eines Patienten 15. Der Patientenaufnahmebereich 14 ist im vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient 15 kann mittels einer Patientenlagerungsvorrichtung 16 des Magnetresonanzgeräts 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf.

Das Magnetresonanzgerät 10 weist weiterhin eine Gradientenspule 18 zum Erzeugen von magnetischen Gradientenfeldern auf, welche für eine Ortskodierung während einer Magnetresonanzmessung verwendet wird. Die Gradientenspule 18 wird mittels einer Gradientensteuereinheit 19 des Magnetresonanzgeräts 10 angesteuert.

Das Magnetresonanzgerät 10 kann weiterhin eine Hochfrequenzantenne umfassen, welche im vorliegenden Ausführungsbeispiel als fest in das Magnetresonanzgerät 10 integrierte Körperspule 20 ausgebildet ist. Die Körperspule 20 ist zu einer Anregung von Kernspins ausgelegt, welche sich in dem von dem Hauptmagneten 12 erzeugten Hauptmagnetfeld 13 befinden. Die Körperspule 20 wird von einer Hochfrequenzeinheit 21 des Magnetresonanzgeräts 10 angesteuert und strahlt hochfrequente Anregungspulse in eine Bildaufnahmeregion ein, die im Wesentlichen von dem Patientenaufnahmebereich 14 des Magnetresonanzgeräts 10 gebildet ist. Die Körperspule 20 kann als eine Empfangseinheit des Magnetresonanzgeräts 10 ausgestaltet sein, welche dazu ausgebildet ist, Magnetresonanzsignale aus dem Patientenaufnahmebereich 14 zu empfangen.

Zu einer Steuerung des Hauptmagneten 12, der Gradientensteuereinheit 19 und der Hochfrequenzeinheit 21 weist das Magnetresonanzgerät 10 eine Steuereinheit 22 auf. Die Steuereinheit 22 ist dazu ausgebildet, eine Durchführung einer Bildgebungssequenz der Bildgebungsuntersuchung, wie z. B. einer GRE (gradient echo) Sequenz, einer TSE (turbo spin echo) Sequenz oder einer UTE (ultra-short echo time) Sequenz, zu steuern. Zudem umfasst die Steuereinheit 22 eine Recheneinheit 28 zu einer Auswertung von Magnetresonanzsignalen, die während einer Magnetresonanzuntersuchung erfasst werden.

Des Weiteren umfasst das Magnetresonanzgerät 10 eine Benutzerschnittstelle 23, welche eine Signalverbindung mit der Steuereinheit 22 aufweist. Steuerinformationen, wie z. B. Bildgebungsparameter der Magnetresonanzuntersuchung, aber auch rekonstruierte Bilddaten einer Körperregion 31 des Patienten 15, sowie eine Hilfestellung zur Anpassung eines Bildgebungsparameters, können auf einer Ausgabeeinheit 24 der Benutzerschnittstelle 23 für einen Nutzer angezeigt werden. Die Ausgabeeinheit 24 kann hierfür beispielsweise einen oder mehrere Monitore umfassen. Die Ausgabeeinheit 24 kann insbesondere dazu ausgelegt sein, eine grafische Benutzeroberfläche mit Bilddaten der Körperregion 31 des Patienten und der Hilfestellung zur Anpassung des Bildgebungsparameters bereitzustellen. Vorzugsweise weist die Benutzerschnittstelle 23 eine Eingabeeinheit 25 auf, welche dem Nutzer ein Anpassen von Bildgebungsparametern, insbesondere eines Parameters eines Bildgebungsbereichs, ermöglicht. Die Eingabeeinheit 25 kann dazu ausgebildet sein, dem Nutzer eine Anpassung einer Dimension, einer Ausrichtung und/oder einer Position eines grafischen Objekts, welches den Bildgebungsbereich repräsentiert, in Abhängigkeit der Bilddaten der Körperregion 31 des Patienten 15 und der Hilfestellung zur Anpassung des Bildgebungsbereichs zu ermöglichen.

Die Recheneinheit 28 ist in dem vorliegenden Beispiel mittels einer Signalverbindung mit einer Speichereinheit 29 des Magnetresonanzgeräts 10 verbunden. Optional kann die Recheneinheit 28 auch mittels einer Signalverbindung mit einer Cloud 30 verbunden sein. Die Recheneinheit 28 kann dazu ausgebildet sein, Daten wie Patienteninformationen, Bilddaten, Localizer-Bilddaten, Magnetresonanzbilder, Röntgenbilder oder dergleichen, auf der Speichereinheit 29 und/oder der Cloud 30 zu speichern und/oder entsprechende Daten von der Speichereinheit 29 und/oder der Cloud 30 mittels einer geeigneten Schnittstelle (nicht dargestellt) abzurufen. Es ist vorstellbar, dass die Recheneinheit 28 dazu ausgebildet ist, eine Patienteninformation des Patienten 15 von der Cloud 30 und/oder der Speichereinheit 29 zu beziehen. Es ist ebenso vorstellbar, dass die Recheneinheit 28 dazu ausgebildet ist, eine Information über die Körperregion 31 des Patienten 15 in Abhängigkeit einer Patienteninformation, insbesondere eines Namen und/oder einer anderen Identifikation, von der Cloud 30 und/oder der Speichereinheit 29 zu beziehen.

In einer bevorzugten Ausführungsform weist das Magnetresonanzgerät 10 eine Dentalspule 26 auf, welche in einer anwendungsgemäßen Position an der Kieferregion 31 und/oder in einer Mundhöhle des Patienten 15 positioniert ist. Die Dentalspule 26 kann ein Antennenelement (nicht gezeigt) aufweisen, welche dazu ausgebildet ist, Magnetresonanzsignale der Kieferregion und/oder der Zahnregion des Patienten 15 zu erfassen und an die Recheneinheit 28 und/oder die Steuereinheit 22 übermitteln.

Die Dentalspule 26 weist vorliegend eine elektrische Anschlussleitung 27 auf, welche eine Signalverbindung mit der Hochfrequenzeinheit 21 bereitstellt. In einer bevorzugten Ausführungsform ist die Dentalspule 26 dazu ausgebildet, Kernspins in der Kieferregion 31 des Patienten 15 anzuregen. Die Dentalspule 26 kann hierfür von der Hochfrequenzeinheit 21 angesteuert werden. In einem Beispiel ist die Dentalspule 26 als eine Maske ausgeführt, welche in einer anwendungsgemäßen Position an einer Hautoberfläche der Kieferregion 31 des Patienten 15 positioniert ist. Es ist jedoch ebenso vorstellbar, dass die Dentalspule 26 mechanisch mit einem Beißelement verbunden ist, welches in einer anwendungsgemäßen Position an einem Zahnbogen, insbesondere entlang einer Okklusionsebene, des Patienten 15 positioniert ist.

Das erfindungsgemäße Magnetresonanzgerät 10 kann selbstverständlich weitere Komponenten umfassen, welche Magnetresonanzgeräte üblicherweise aufweisen. Es ist ebenso vorstellbar, dass das Magnetresonanzgerät 10 statt des zylinderförmigen Aufbaus einen C-förmigen, einen dreieckigen oder einen asymmetrischen Aufbau der Magnetfeld-erzeugenden Komponenten aufweist. Das Magnetresonanzgerät 10 kann insbesondere dazu ausgebildet ein, eine Magnetresonanzuntersuchung eines stehenden oder sitzenden Patienten 15 durchzuführen.

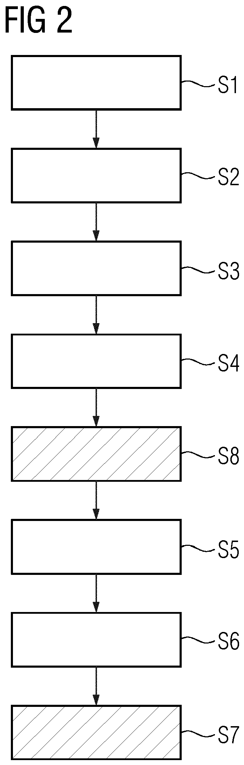

In Fig. 2 ist ein Ablaufdiagramm einer Ausführungsform eines erfindungsgemäßen Verfahrens zur Bestimmung eines Bildgebungsprotokolls zur Erfassung von Bilddaten einer Körperregion eines Patienten mittels einer Bildgebungsvorrichtung 1 dargestellt. Der Ablauf der beispielhaften Ausführungsform des Verfahrens wird im Folgenden anhand eines Magnetresonanzgeräts 10 erläutert. Selbstverständlich kann das erfindungsgemäße Verfahren auch mittels einer anderen Bildgebungsvorrichtung gemäß einer oben beschriebenen Ausführungsform ausgeführt werden.

In dem Schritt S1 werden Informationen über die Körperregion des Patienten erfasst. Es ist vorstellbar, dass die Information über die Körperregion des Patienten von einem Nutzer des Magnetresonanzgeräts 10 mittels der Benutzerschnittstelle 23 eingegeben werden. Die Benutzerschnittstelle 23 kann hierfür eine beliebige Eingabeeinheit 25 umfassen, welche eine Interaktion des Nutzers mit einer grafischen Benutzerschnittstelle ermöglicht. Vorzugsweise weist die Eingabeeinheit 25 eine Tastatur, eine Maus und/oder einen Touchscreen auf. Die von dem Nutzer eingegebene Information über die Körperregion des Patienten 15 kann anschließend mittels der Steuereinheit 22 und/oder der Recheneinheit 28 des Magnetresonanzgeräts 10 erfasst und verarbeitet werden.

Es ist ebenso vorstellbar, dass die Information über die Körperregion des Patienten in Abhängigkeit einer Patienteninformation von einem radiologischen Informationssystem, einem Krankenhausinformationssystem, einer Netzwerk-Speichereinheit, einer lokalen Speichereinheit 29 und/oder einer Cloud 30 erfasst oder abgefragt werden.

In dem Schritt S2 wird eine erste Bildgebungsuntersuchung zur Erfassung von Bilddaten der Körperregion in Abhängigkeit der Information über die Körperregion durchgeführt.

Vorzugsweise ist die Steuereinheit 22 und/oder die Recheneinheit 28 dazu ausgebildet, in Abhängigkeit der Information über die Körperregion des Patienten 15 ein erstes Bildgebungsprotokoll oder einen ersten Bildgebungsbereich, welcher die erste Bildgebungsuntersuchung charakterisieren, zu bestimmen. Das Magnetresonanzgerät 10 kann insbesondere dazu ausgebildet sein, die erste Bildgebungsuntersuchung in Abhängigkeit des ersten Bildgebungsbereichs und/oder des ersten Bildgebungsprotokolls auszuführen. Beispielsweise können das erste Bildgebungsprotokoll und/oder der erste Bildgebungsbereich automatisch in Abhängigkeit der Information über die Körperregion mittels der Steuereinheit 22 und/oder Recheneinheit 28 ausgewählt oder von einem Nutzer des Magnetresonanzgeräts 10 mittels der Benutzerschnittstelle 23 vorgegeben werden. Es ist weiterhin vorstellbar, dass das erste Bildgebungsprotokoll und/oder der erste Bildgebungsbereich ein Teil einer Information über die Körperregion des Patienten 15 darstellen.

Die erste Bildgebungsuntersuchung kann dazu ausgebildet sein, niedrig-aufgelöste Magnetresonanzdaten oder Bilddaten mit niedriger Qualität von der Körperregion des Patienten zu erfassen. Insbesondere kann die erste Bildgebungsuntersuchung eine Localizer-Messung darstellen. Es ist ebenso vorstellbar, dass die erste Bildgebungsuntersuchung eine Bildgebungsuntersuchung oder Magnetresonanzuntersuchung umfasst, welche eine Geschwindigkeit eines Erfassens von Bilddaten gegenüber einer Qualität und/oder einer Auflösung der Bilddaten priorisiert.

Der Schritt S3 umfasst ein Bereitstellen der Bilddaten und einer Eingabeoption zur Anpassung eines Parameters eines Bildgebungsbereichs. Vorzugsweise werden die in dem Schritt S2 erfassten Bilddaten der Körperregion des Patienten dem Nutzer des Magnetresonanzgeräts 10 mittels der Ausgabeeinheit 25 bereitgestellt. Das Bereitstellen der Bilddaten kann insbesondere ein Anzeigen der Bilddaten auf einem Monitor der Benutzerschnittstelle 23 umfassen.

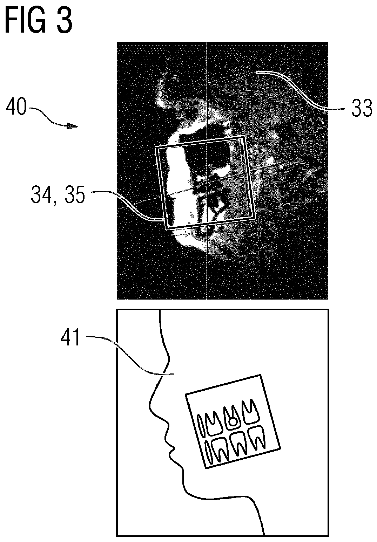

Vorzugsweise stellen die bereitgestellten Bilddaten der Körperregion des Patienten und die Eingabeoption zur Anpassung des Parameters des Bildgebungsbereichs ein Teil einer Ausgabe 40 der Benutzerschnittstelle 23 dar (siehe Figuren 3 bis 5), welche dem Nutzer ein Anpassen einer Magnetresonanzuntersuchung, insbesondere eines Parameters eines Bildgebungsbereichs für eine weitere Bildgebungsuntersuchung, ermöglicht.

Eine Eingabeoption zur Anpassung des Parameters des Bildgebungsbereichs kann beispielsweise eine textbasierte Eingabemaske und/oder ein textbasiertes Eingabefenster umfassen. Es ist jedoch ebenso vorstellbar, dass die Eingabeoption zur Anpassung des Parameters des Bildgebungsbereichs eine grafische Repräsentation des Bildgebungsbereichs oder ein grafisches Objekt umfasst, welches von dem Nutzer angepasst werden kann. Beispielsweise kann der Nutzer eine Dimension, eine Ausrichtung und/oder eine Position der Repräsentation des Bildgebungsbereichs anpassen, um den Parameter des Bildgebungsbereichs anzupassen. Vorzugsweise sind die Steuereinheit 22 und/oder die Recheneinheit 28 des Magnetresonanzgeräts 10 dazu ausgebildet, die Anpassung des Parameters anhand der vom Nutzer geänderten Repräsentation des Bildgebungsbereichs abzuleiten oder zu ermitteln.

Der Schritt S4 des erfindungsgemäßen Verfahrens umfasst ein Bereitstellen einer Hilfestellung zur Anpassung des Bildgebungsbereichs in Abhängigkeit der Information über die Körperregion.

In einer bevorzugten Ausführungsform umfasst das Bereitstellen der Hilfestellung zur Anpassung des Bildgebungsbereichs ein Ausgeben einer textbasierten Anleitung und/oder einer grafischen Anleitung. Die grafische Anleitung kann insbesondere eine Repräsentation der Körperregion 31 des Patienten 15 und des Bildgebungsbereichs umfassen (siehe Figuren 3 bis 5). Die Hilfestellung zur Anpassung des Bildgebungsbereichs kann einen Nutzer des Magnetresonanzgeräts 10 dazu befähigen, den Bildgebungsbereich für eine zweite Bildgebungsuntersuchung anzupassen.

Vorzugsweise wird die Hilfestellung zur Anpassung des Bildgebungsbereichs als eine grafische Anleitung zeitsynchron zu einer Repräsentation des vorläufigen Bildgebungsbereichs und den Bilddaten der Körperregion 31 des Patienten 15 bereitgestellt. Dadurch wird der Nutzer dazu befähigt, den Bildgebungsbereich in Abhängigkeit einer Differenz zwischen dem vorläufigen Bildgebungsbereich, der Hilfestellung zur Anpassung des Bildgebungsbereichs, aber auch einer Gestalt einer individuellen anatomischen Struktur der Körperregion 31 des Patienten 15, anzupassen.

In dem optionalen Schritt S8 erfolgt ein Bestimmen eines vorläufigen Bildgebungsbereichs in Abhängigkeit der Bilddaten, wobei das Bereitstellen der Eingabeoption zur Anpassung des Parameters des Bildgebungsbereichs eine Überlagerung einer Repräsentation des vorläufigen Bildgebungsbereichs mit den Bilddaten der Körperregion 31 des Patienten 15 umfasst.

Vorzugsweise wird der vorläufige Bildgebungsbereich automatisch mittels der Steuereinheit 22 und/oder der Recheneinheit 28 des Magnetresonanzgeräts 10 in Abhängigkeit der Bilddaten der Körperregion 31 des Patienten 15 ermittelt. Es ist vorstellbar, dass die Steuereinheit 22 und/oder die Recheneinheit 28 einen Bildverarbeitungsalgorithmus umfassen, welcher dazu ausgebildet ist, die Körperregion 31 oder einen diagnostisch relevanten Abschnitt der Körperregion 31 des Patienten 15 in den Bilddaten zu identifizieren und den an die Körperregion 31 oder den diagnostisch relevanten Abschnitt der Körperregion 31 des Patienten 15 angepassten, vorläufigen Bildgebungsbereich zu ermitteln und/oder bereitzustellen.

In einer bevorzugten Ausführungsform umfasst der vorläufige Bildgebungsbereich eine Repräsentation eines Bildgebungsbereichs, insbesondere ein grafisches Objekt, welches den Bilddaten überlagert in anwendungsgemäßer Weise relativ zu der Körperregion 31 des Patienten 15 angeordnet ist. Das grafische Objekt kann eine Position, eine Ausrichtung und/oder eine Dimension des Bildgebungsbereichs angeben, welche mittels der Benutzerschnittstelle 23 interaktiv von dem Nutzer bei der Anpassung des Bildgebungsbereichs geändert werden können.

In einem weiteren Schritt S5 wird ein angepasster Bildgebungsbereich erfasst. Vorzugsweise wird ein durch den Nutzer mittels der Benutzerschnittstelle 23 angepasster Bildgebungsbereich von der Steuereinheit 22 und/oder der Recheneinheit 28 erfasst. Beispielsweise können die Steuereinheit 22 und/oder die Recheneinheit 28 dazu ausgebildet sein, eine durch den Nutzer geänderte Position, Dimension und/oder Ausrichtung eines vorläufigen Bildgebungsbereichs und/oder eines grafischen Objekts, welches den Bildgebungsbereich repräsentiert, zu erfassen und daraus den angepassten Bildgebungsbereich abzuleiten. Es ist vorstellbar, dass das Erfassen des angepassten Bildgebungsbereichs ein Erfassen einer Eingabe einer Maus, einer Tastatur und/oder einer Touch-Eingabe eines Touchscreens umfasst.

Das Erfassen des angepassten Bildgebungsbereichs kann insbesondere ein Speichern des angepassten Bildgebungsbereichs auf der Speichereinheit 29, einem Cloud-Speicher 30, einem Netzwerk-Speicher oder dergleichen umfassen.

In einem Schritt S6 wird das Bildgebungsprotokoll für eine zweite Bildgebungsuntersuchung in Abhängigkeit des angepassten Bildgebungsbereichs bestimmt. Die Bestimmung des Bildgebungsprotokolls für die zweite Bildgebungsuntersuchung kann insbesondere automatisch auf Basis des angepassten Bildgebungsbereichs erfolgen.

Vorzugsweise wird das Bestimmen des Bildgebungsprotokolls für die zweite Bildgebungsuntersuchung automatisch mittels der Steuereinheit 22 und/oder der Recheneinheit durchgeführt. Es ist vorstellbar, dass die Recheneinheit 28 einen Algorithmus umfasst, welcher dazu ausgebildet ist, das Bildgebungsprotokoll für die zweite Bildgebungsuntersuchung in Abhängigkeit des angepassten Bildgebungsbereichs zu ermitteln. Beispielsweise kann der Algorithmus dazu ausgebildet sein, eine durch den Nutzer vorgenommene Veränderung einer Dimension, einer Position und/oder einer Ausrichtung des vorläufigen Bildgebungsbereichs zu erfassen und in ein Bildgebungsprotokoll für die zweite Bildgebungsuntersuchung zu übersetzen. Die Steuereinheit 22 und/oder die Recheneinheit 28 können jedoch ebenso dazu ausgebildet sein, das Bildgebungsprotokoll anhand von numerischen Daten und/oder einer textbasierten Eingabe des Nutzers zu ermitteln.

In einem optionalen Schritt S7 wird eine zweite Bildgebungsuntersuchung zum Erfassen von weiteren Bilddaten der Körperregion 31 des Patienten 15 in Abhängigkeit des Bildgebungsprotokolls durchgeführt. Vorzugsweise wird die zweite Bildgebungsuntersuchung mittels der Bildgebungsvorrichtung 1, insbesondere des Magnetresonanzgeräts 10, durchgeführt.

Zur Durchführung der zweiten Bildgebungsuntersuchung kann der Patient 15, wie in Fig. 1 gezeigt, anwendungsgemäß in dem Patientenaufnahmebereich 14 des Magnetresonanzgeräts 10 positioniert sein. Die zweite Bildgebungsuntersuchung ist beispielsweise eine Magnetresonanzuntersuchung einer Zahnregion oder eines Abschnitts einer Zahnregion des Patienten 15. Vorzugsweise ist eine Auflösung mittels der zweiten Bildgebungsuntersuchung erfasster weiterer Bilddaten höher als eine Auflösung der Bilddaten. Es ist ebenso vorstellbar, dass ein diagnostisch relevanter Abschnitt der Körperregion 31 des Patienten 15 in den weiteren Bilddaten besser zentriert und/oder fokussiert ist als in den Bilddaten der ersten Bildgebungsuntersuchung.

In Fig. 3 ist eine schematische Repräsentation einer Hilfestellung 41 zur Anpassung des Bildgebungsbereichs gemäß einer Ausführungsform des erfindungsgemäßen Verfahrens dargestellt. Die beispielhafte Darstellung in Fig. 3 kann insbesondere eine Ausgabe 40 der Ausgabeeinheit 25 und/oder der Benutzerschnittstelle 23 für einen Nutzer der Bildgebungsvorrichtung 1 repräsentieren.

Das Bereitstellen der Bilddaten 33 und der Eingabeoption 34 zur Anpassung des Parameters des Bildgebungsbereichs 35 gemäß des Schritts S3 umfasst vorliegend ein Gegenüberstellen der Bilddaten 33 des Patienten 15 mit der Hilfestellung 41 zur Anpassung des Bildgebungsbereichs. Die Hilfestellung 41 zur Anpassung des Bildgebungsbereichs umfasst insbesondere eine grafische Anleitung mit einer Repräsentation der Körperregion 31 des Patienten 15 und einer Repräsentation eines gewünschten oder idealen Bildgebungsbereichs.

Vorzugsweise ist eine Repräsentation des vorläufigen Bildgebungsbereichs 35 den Bilddaten 33 der Körperregion 31 des Patienten 15 überlagert dargestellt. Die Repräsentation des vorläufigen Bildgebungsbereichs 35 kann gleichzeitig ein grafisches Objekt darstellen, welches dem Nutzer ein Anpassen des Bildgebungsbereichs 35 ermöglicht. Der vorläufige Bildgebungsbereich 35 kann dem Nutzer in Abhängigkeit der Information über die Körperregion 31 automatisch vorgeschlagen werden und auf einem gewünschten oder idealen Bildgebungsbereich für eine spezifische Körperregion oder einem spezifischen Abschnitt einer Körperregion beruhen. Beispielsweise können die Steuereinheit 22 und/oder die Recheneinheit 28 dazu ausgebildet sein, den vorläufigen Bildgebungsbereich 35 in Abhängigkeit der Information über die Körperregion 31 des Patienten 15 und/oder der Bilddaten der Körperregion 31 des Patienten 15 auszuwählen oder zu ermitteln.

Das Bereitstellen der Bilddaten 33 und der Eingabeoption zur Anpassung des Parameters des Bildgebungsbereichs 35 umfasst vorliegend ein Ausgeben einer auf den Bilddaten 33 basierenden Schnittansicht der Körperregion 31 des Patienten 15. Sowohl die Bilddaten 33 der Körperregion 31 des Patienten 15 als auch die grafische Anleitung (der Hilfestellung zur Anpassung des Bildgebungsbereichs 35) zeigen vorliegend jeweils eine Schnittansicht des Kopfes oder der Zahnregion des Patienten 15. Die Bezugsebenen der Schnittansichten der Bilddaten 33 der Körperregion 31 des Patienten 15 als auch der grafischen Anleitung sind in dem gezeigten Beispiel parallel zu einer Sagittalebene des Patienten 15 ausgerichtet.

Vorzugsweise umfasst die Eingabeoption 34 zur Anpassung des Parameters des Bildgebungsbereichs 35 oder der vorläufige Bildgebungsbereich 35 ein grafisches Objekt, welches es dem Nutzer durch Manipulation einer Dimension, einer Position und/oder einer Ausrichtung des grafischen Objekts erlaubt, den Bildgebungsbereich 35 in Abhängigkeit der Hilfestellung 41 zur Anpassung des Bildgebungsbereichs anzupassen.

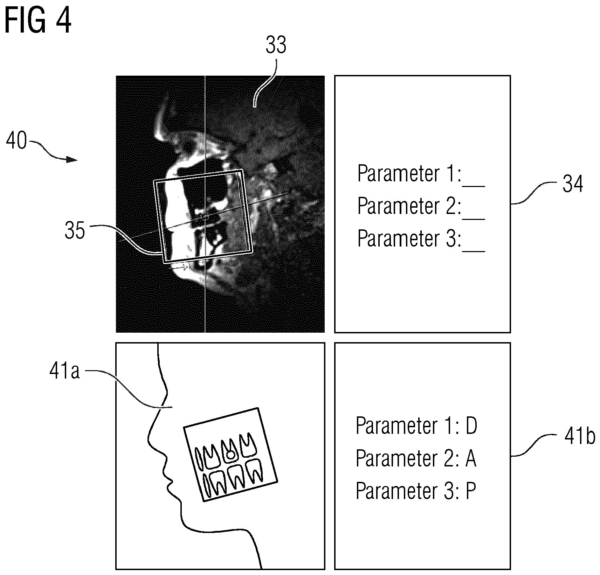

In Fig. 4 ist eine schematische Repräsentation einer Hilfestellung 41 zur Anpassung des Bildgebungsbereichs analog gemäß einer weiteren Ausführungsform des erfindungsgemäßen Verfahrens dargestellt. Die beispielhafte Darstellung in Fig. 3 kann insbesondere eine Ausgabe 40 der Ausgabeeinheit 25 und/oder der Benutzerschnittstelle 23 für einen Nutzer der Bildgebungsvorrichtung 1 repräsentieren.

Im Unterschied zu der in Fig. 3 gezeigten Ausführungsform, umfasst die Eingabeoption 34 zur Anpassung des Parameters des Bildgebungsbereichs 35 ein Textfeld zur Eingabe von Parametern des Bildgebungsbereichs 35. Beispielsweise können der Parameter 1 eine Dimension D, Parameter 2 eine Ausrichtung A und Parameter 3 eine Position des Bildgebungsbereichs 35 definieren. Daneben ist auch eine Eingabe weiterer Parameter, welche für die jeweilige Bildgebungsvorrichtung 1 relevant sind, möglich. Es ist weiterhin vorstellbar, dass die Eingabeoption 34 zur Anpassung des Parameters des Bildgebungsbereichs 35 wie in Fig. 3 gezeigt, ein grafisches Objekt umfasst, welches dem Nutzer eine grafische Anpassung des Bildgebungsbereichs 35 gemäß einer oben beschriebenen Ausführungsform ermöglicht. Vorzugsweise werden die Parameter 1 bis 3 in Abhängigkeit einer Manipulation des grafischen Objekts durch den Nutzer aktualisiert.

In dem in Fig. 4 gezeigten Beispiel umfasst die Hilfestellung 41 zur Anpassung des Bildgebungsbereichs 35 sowohl eine grafische Anleitung 41a als auch eine textbasierte Anleitung 41b. Die textbasierte Anleitung 41b kann eine gewünschte oder ideale Parametrierung des Bildgebungsbereichs 35 für eine spezifische Körperregion oder einen spezifischen Abschnitt einer Körperregion des Patienten 35 umfassen. Vorliegend stellt die textbasierte Anleitung 41b dem Nutzer eine gewünschte Dimension D, eine gewünschte Ausrichtung A und eine gewünschte Position P des Bildgebungsbereichs 35 für einen linken Abschnitt eines oberen Zahnbogens des Patienten 15 bereit. Der Nutzer wird entsprechend dazu befähigt, den Bildgebungsbereich 35 in Abhängigkeit der Hilfestellung 41 zur Anpassung des Bildgebungsbereichs 35 und den Bilddaten 33 mit der tatsächlichen Gestalt der anatomischen Strukturen des Patienten 15, anzupassen.

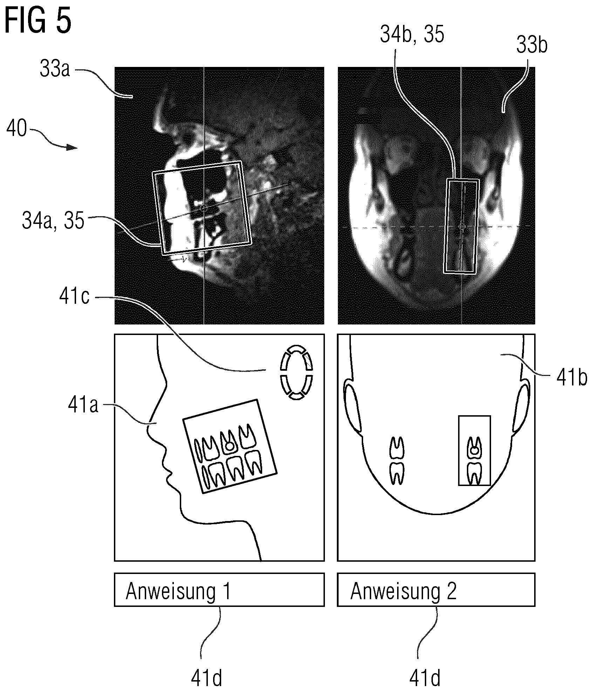

Die Fig. 5 zeigt eine weitere schematische Repräsentation einer Hilfestellung 41 zur Anpassung des Bildgebungsbereichs gemäß einer bevorzugten Ausführungsform des erfindungsgemäßen Verfahrens. Analog zu den in den Figuren 3 und 4 gezeigten Ausführungsformen werden die Bilddaten 33 der Körperregion 31 des Patienten 15 und die Eingabeoption 34 zur Anpassung des Parameters des Bildgebungsbereichs 35 mit einer grafischen Anleitung zur Anpassung des Bildgebungsbereichs gegenübergestellt.

In dem Beispiel der Fig. 5 umfasst das Bereitstellen der Hilfestellung 41 zur Anpassung des Bildgebungsbereichs 35 ein Ausgeben einer grafischen Anleitung mit Repräsentationen der Körperregion 31 des Patienten 15 und einem gewünschten oder idealen Bildgebungsbereich. Die grafische Anleitung umfasst vorliegend zwei Schnittansichten 41a und 41b der Körperregion 31 des Patienten 15, wobei die zwei Schnittansichten 41a und 41b entlang unterschiedlicher Bezugsebenen ausgerichtet sind. In dem gezeigten Beispiel ist die Bezugsebene der Schnittansicht 41a parallel zu einer Sagittalebene des Patienten 15 ausgerichtet, während die Bezugsebene der Schnittansicht 41b parallel zu einer Frontalebene des Patienten 15 ausgerichtet ist.

Vorzugsweise umfasst das Bereitstellen der Bilddaten 33 und der Eingabeoption 34 zur Anpassung des Parameters des Bildgebungsbereichs 35 weiterhin ein Ausgeben von zwei auf den Bilddaten basierenden Schnittansichten 33a und 33b der Körperregion des Patienten, wobei je eine Bezugsebene der zwei auf den Bilddaten basierenden Schnittansichten 33a und 33b der Körperregion 31 des Patienten 15 parallel zu je einer Bezugsebene der zwei Schnittansichten 41a und 41b der grafischen Anleitung ausgerichtet ist.

Die auf den Bilddaten basierenden Schnittansichten 33a und 33b der Körperregion 31 des Patienten 15 enthalten je eine Eingabeoption 34a und 34b zur Anpassung des Parameters des Bildgebungsbereichs 35. Die Eingabeoptionen 34a und 34b sind vorliegend als grafische Objekte ausgestaltet, welche gemäß einer oben beschriebenen Ausführungsform durch den Nutzer manipulierbar sind. Vorzugsweise werden eine Dimension, eine Ausrichtung und/oder eine Position der grafischen Objekte der Eingabeoptionen 34a und 34b zur Anpassung des Parameters des Bildgebungsbereichs 35 aktualisiert, wenn der Nutzer eine Anpassung des Bildgebungsbereichs 35 vorgenommen hat.

Da eine links-rechts-Positionierung des Bildgebungsbereichs 35 relativ zu der Zahnregion des Patienten 15 allein aus der Schnittansicht 41a unklar sein kann, umfasst die Schnittansicht 41a vorzugsweise eine Übersichtskarte 41c der Körperregion 31 des Patienten 15, in welcher ein oder mehrere diagnostisch relevante Abschnitte der Zahnregion des Patienten 15 markiert oder hervorgehoben sind. Die Übersichtskarte 41c kann automatisch mittels der Steuereinheit 22 und/oder der Recheneinheit 28 generiert und zusammen mit der Hilfestellung 41 zur Anpassung des Bildgebungsbereichs 35 ausgegeben werden. Es ist vorstellbar, dass auch die Hilfestellungen 41 zur Anpassung des Bildgebungsbereichs der Ausführungsformen der Figuren 3 und 4 eine entsprechende Übersichtskarte 41c umfassen.

Es ist vorstellbar, dass die Übersichtskarte 41c in dem Schritt S1 dem Nutzer als Eingabeoption bereitgestellt wird, um die Information über die Körperregion 31 des Patienten 15 zu erfassen.

In einer Ausführungsform des erfindungsgemäßen Verfahrens umfasst das Bereitstellen der Hilfestellung 41 zur Anpassung des Bildgebungsbereichs in Abhängigkeit der Körperregion 31 des Patienten 15 ferner ein Ausgeben einer textbasierten Anleitung. In dem in Fig. 5 gezeigten Beispiel enthält die textbasierte Anleitung zwei Anweisungen 41d, welche Instruktionen zum Ablauf der Anpassung des Bildgebungsbereichs 35 umfassen. Beispielsweise weist die Anweisung 1 den Nutzer dazu an, den Bildgebungsbereich 35 über einer diagnostisch relevanten Zahngruppe, welche mittels der zweiten Bildgebungsuntersuchung gescannt werden soll, zu positionieren. Die Anweisung 2 kann den Nutzer dazu anweisen, den Bildgebungsbereich 35 relativ zu einem diagnostisch relevanten Abschnitt der Zahngruppe auszurichten.

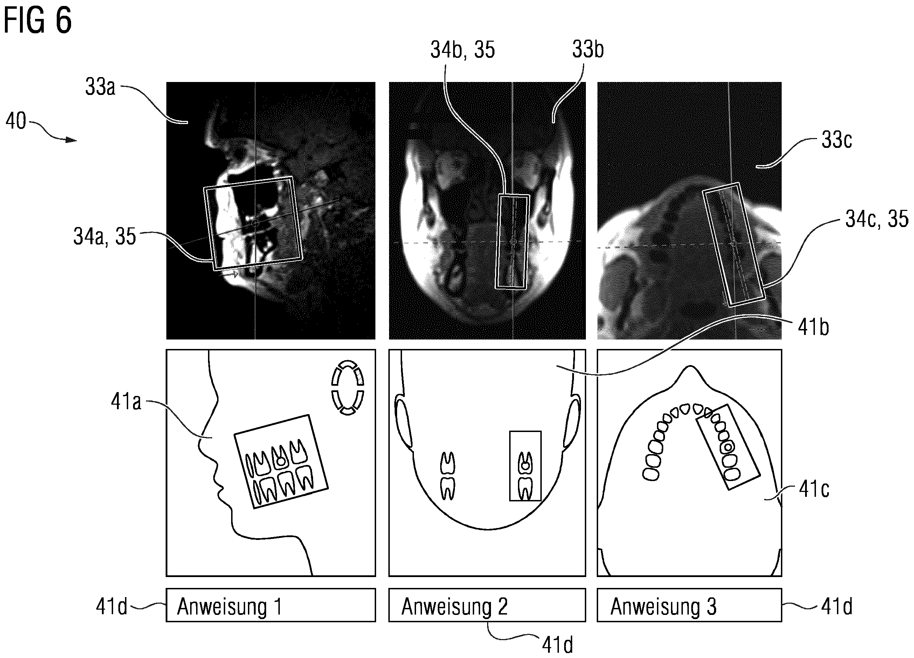

Die Fig. 6 zeigt eine weitere schematische Repräsentation einer Hilfestellung 41 zur Anpassung des Bildgebungsbereichs gemäß einer bevorzugten Ausführungsform des erfindungsgemäßen Verfahrens. Analog zu der in Fig. 5 gezeigten Ausführungsformen werden die Bilddaten 33 der Körperregion 31 des Patienten 15 und die Eingabeoption 34 zur Anpassung des Parameters des Bildgebungsbereichs 35 mit einer grafischen Anleitung zur Anpassung des Bildgebungsbereichs gegenübergestellt.

In dem Beispiel der Fig. 6 umfasst das Bereitstellen der Hilfestellung 41 zur Anpassung des Bildgebungsbereichs ein Ausgeben einer grafischen Anleitung mit Repräsentationen der Körperregion 31 des Patienten 15 und einem gewünschten oder idealen Bildgebungsbereich. Die grafische Anleitung umfasst vorliegend drei Schnittansichten 41a, 41b und 41c der Körperregion 31 des Patienten 15, welche entlang unterschiedlicher Bezugsebenen ausgerichtet sind. In dem gezeigten Beispiel ist die Bezugsebene der Schnittansicht 41a parallel zu einer Sagittalebene des Patienten 15 ausgerichtet. Die Bezugsebene der Schnittansicht 41b ist parallel zu einer Frontalebene des Patienten 15 ausgerichtet und die Bezugsebene der Schnittansicht 41c ist parallel zu einer Transversalebene oder Okklusionsebene des Patienten ausgerichtet.

Vorzugsweise umfasst das Bereitstellen der Bilddaten 33 und der Eingabeoption 34 zur Anpassung des Parameters des Bildgebungsbereichs 35 weiterhin ein Ausgeben von drei auf den Bilddaten basierenden Schnittansichten 33a, 33b und 33c der Körperregion 31 des Patienten 15, wobei je eine Bezugsebene der drei auf den Bilddaten 33 basierenden Schnittansichten 33a, 33b und 33c der Körperregion des Patienten parallel zu je einer Bezugsebene der drei Schnittansichten 41a, 41b und 41c der grafischen Anleitung ausgerichtet ist.

Die auf den Bilddaten basierenden Schnittansichten 33a, 33b und 33c der Körperregion des Patienten enthalten jeweils eine Eingabeoption 34a, 34b und 34c zur Anpassung eines Parameters des Bildgebungsbereichs 35. Die Eingabeoptionen 34a, 34b und 34c sind vorliegend als grafische Objekte ausgestaltet, welche gemäß einer oben beschriebenen Ausführungsform durch den Nutzer manipuliert werden können. Vorzugsweise werden eine Dimension, eine Ausrichtung und/oder eine Position der grafischen Objekte der Eingabeoptionen 34a, 34b und 34c aktualisiert, wenn der Nutzer eine entsprechende Anpassung des Bildgebungsbereichs 35 vornimmt.

Das Bereitstellen der Hilfestellung 41 zur Anpassung des Bildgebungsbereichs 35 kann weiterhin ein Ausgeben einer oder mehrerer textbasierter Anleitungen 41d umfassen, welche beispielsweise Instruktionen zum Ablauf der Anpassung des Bildgebungsbereichs 35 enthalten. Die textbasierten Anleitungen 41d können den Nutzer bei der Anpassung des Bildgebungsbereich 35 mittels der Eingabeoptionen 34a, 34b und 34c hinsichtlich zu beachtender Randbedingungen informieren. Beispielsweise können die textbasierten Anleitungen 41d den Nutzer über eine vorteilhafte Reihenfolge der Anpassung des Bildgebungsbereichs 35 mittels der Eingabeoptionen 34a, 34b und 34c in den auf den Bilddaten basierenden Schnittansichten 33a, 33b und 33c der Körperregion 31 des Patienten 15 informieren. Insbesondere können die textbasierten Anleitungen 41d den Nutzer darüber informieren, welcher Parameter des Bildgebungsbereichs 35 in Relation zu welcher auf den Bilddaten basierenden Schnittansicht 33 angepasst wird, um einen zeiteffizienten Verfahrensablauf zu erreichen und/oder Fehler bei der Anpassung des Bildgebungsbereichs 35 zu vermeiden.

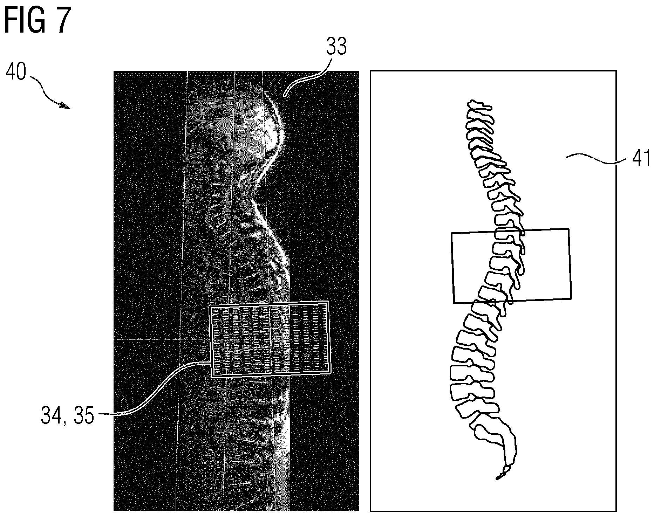

In den Figuren 3 bis 6 ist die Körperregion 31 des Patienten 15 eine Kieferregion und/oder eine Zahnregion des Patienten 15. Die Fig. 7 zeigt eine Ausführungsform des erfindungsgemäßen Verfahrens, bei welcher das Bereitstellen der Bilddaten 33 der Körperregion 31 des Patienten 15 und der Hilfestellung 41 zur Anpassung des Bildgebungsbereichs 35 eine Wirbelsäule oder einen Abschnitt einer Wirbelsäule des Patienten 15 betreffen. Analog zu den oben beschriebenen Ausführungsformen werden die Bilddaten 33 der Wirbelsäule des Patienten 15 einer grafischen Anleitung 41 zur Anpassung des Bildgebungsbereichs 35 gegenübergestellt. Dabei ist eine Repräsentation des vorläufigen Bildgebungsbereichs 35 den Bilddaten 33 der Wirbelsäule des Patienten 15 überlagert dargestellt. Die Repräsentation des vorläufigen Bildgebungsbereichs 35 kann gleichzeitig ein grafisches Objekt 34 umfassen, welches dem Nutzer ein Anpassen des Bildgebungsbereichs 35 ermöglicht.

Selbstverständlich kann das erfindungsgemäße Verfahren zur Bestimmung eines Bildgebungsprotokolls zur Erfassung von Bilddaten beliebiger anderer Körperregionen verwendet werden. Insbesondere kann das erfindungsgemäße Verfahren zur Bestimmung eines Bildgebungsprotokolls zur Erfassung von Bilddaten von Körperregionen mit einer Vielzahl von unterschiedlich ausgerichteten Abschnitten oder Subregionen, wie z. B. einer Hand, einem Fuß, einer Zahnregion, aber auch anderen anatomischen Strukturen und/oder Organen, verwendet werden.

Die hier beschriebenen Ausführungsformen des erfindungsgemäßen Verfahrens und der erfindungsgemäßen Bildgebungsvorrichtung sind als beispielhaft zu verstehen. Wenn nicht im Detail anders erläutert, sind einzelne Ausführungsformen grundsätzlich um Merkmale anderer Ausführungsformen erweiterbar. Insbesondere ist die Reihenfolge der Verfahrensschritte des erfindungsgemäßen Verfahrens als beispielhaft zu verstehen. Die einzelnen Schritte können auch in einer anderen Reihenfolge durchgeführt werden oder sich zeitlich teilweise oder vollständig überschneiden. Beispielsweise können die Schritte des Erfassens der Information über die Körperregion und des Durchführens der ersten Bildgebungsuntersuchung in einer beliebigen Reihenfolge nacheinander oder zumindest teilweise überlappend ablaufen. Gleichermaßen kann das Bereitstellen der Bilddaten und der Eingabeoption zur Anpassung des Parameters des Bildgebungsbereichs sowie das Bereitstellen der Hilfestellung zur Anpassung des Bildgebungsbereichs in Abhängigkeit der Information über die Körperregion in einer beliebigen Reihenfolge nacheinander oder zumindest teilweise überlappend durchgeführt werden.

## Patentansprüche

1. Ein Computer-implementiertes Verfahren zur Bestimmung eines Bildgebungsprotokolls zur Erfassung von Bilddaten (33) einer Körperregion (31) eines Patienten (15) mittels einer Bildgebungsvorrichtung (1), umfassend die Schritte:
• Erfassen (S1) einer Information über die Körperregion (31),
• Durchführen (S2) einer ersten Bildgebungsuntersuchung in Abhängigkeit der Information über die Körperregion (31) und Erfassen von Bilddaten (33) der Körperregion (31),
• Bereitstellen (S3) der Bilddaten (31) und einer Eingabeoption (34) zur Anpassung eines Parameters eines Bildgebungsbereichs (35),
• Bereitstellen (S4) einer Hilfestellung (41) zur Anpassung des Bildgebungsbereichs in Abhängigkeit der Information über die Körperregion (31),
• Erfassen (S5) eines angepassten Bildgebungsbereichs (35), und
• Bestimmen (S6) des Bildgebungsprotokolls für eine zweite Bildgebungsuntersuchung in Abhängigkeit des angepassten Bildgebungsbereichs (35).

2. Das Verfahren nach Anspruch 1, aufweisend den Schritt:
• Bestimmen (S8) eines vorläufigen Bildgebungsbereichs in Abhängigkeit der Bilddaten (33),
wobei das Bereitstellen der Eingabeoption (34) zur Anpassung des Parameters des Bildgebungsbereichs (35) eine Überlagerung einer Repräsentation des vorläufigen Bildgebungsbereichs mit den Bilddaten (33) der Körperregion (31) des Patienten (15) umfasst.

3. Das Verfahren nach Anspruch 2, wobei das Erfassen (S5) des angepassten Bildgebungsbereichs (35) ein Erfassen einer Korrektur des vorläufigen Bildgebungsbereichs umfasst.

4. Das Verfahren nach einem der vorhergehenden Ansprüche, ferner aufweisend den Schritt:
• Durchführen (S7) der zweiten Bildgebungsuntersuchung zum Erfassen von weiteren Bilddaten der Körperregion (31) des Patienten (15) in Abhängigkeit des Bildgebungsprotokolls.

5. Das Verfahren nach einem der vorhergehenden Ansprüche, wobei das Bereitstellen (S4) der Hilfestellung (41) zur Anpassung des Bildgebungsbereichs ein Ausgeben einer textbasierten Anleitung und/oder einer grafischen Anleitung umfasst.

6. Das Verfahren nach Anspruch 5, wobei das Bereitstellen (S4) der Hilfestellung (41) zur Anpassung des Bildgebungsbereichs das Ausgeben einer grafischen Anleitung umfasst und wobei die grafische Anleitung eine Repräsentation der Körperregion (31) und des Bildgebungsbereichs umfasst.

7. Das Verfahren nach Anspruch 6, wobei die grafische Anleitung zumindest zwei Schnittansichten der Körperregion (31) des Patienten (15) umfasst, und wobei die zumindest zwei Schnittansichten der Körperregion (31) des Patienten (15) entlang unterschiedlicher, vorzugsweise orthogonal zueinander ausgerichteter, Bezugsebenen ausgerichtet sind.

8. Das Verfahren nach Anspruch 7, wobei das Bereitstellen (S3) der Bilddaten (33) und der Eingabeoption (34) zur Anpassung des Parameters des Bildgebungsbereichs (35) ein Ausgeben von zumindest zwei auf den Bilddaten (33) basierenden Schnittansichten der Körperregion (31) des Patienten (15) umfasst, und wobei je eine Bezugsebene der zumindest zwei auf den Bilddaten (33) basierenden Schnittansichten der Körperregion (31) des Patienten (15) parallel zu je einer Bezugsebene der zumindest zwei Schnittansichten der Körperregion (31) des Patienten (15) der grafischen Anleitung ausgerichtet ist.

9. Das Verfahren nach einem der Ansprüche 6 bis 8, wobei das Bereitstellen (S3) der Bilddaten (33) und der Eingabeoption (34) zur Anpassung des Parameters des Bildgebungsbereichs (35) ein Gegenüberstellen der Bilddaten (33) mit der grafischen Anleitung umfasst.

10. Das Verfahren nach einem der vorhergehenden Ansprüche, wobei die Körperregion (31) einen Anatomiebereich einer ersten Körperhälfte des Patienten (15) umfasst, welcher im Wesentlichen symmetrisch zu einem Anatomiebereich in einer der ersten Körperhälfte gegenüberliegenden zweiten Körperhälfte des Patienten (15) aufgebaut ist.

11. Das Verfahren nach einem der vorhergehenden Ansprüche, wobei die Körperregion (31) des Patienten (15) eine Kieferregion und/oder eine Zahnregion umfasst.

12. Das Verfahren nach den Ansprüchen 7 und 11, wobei eine Bezugsebene einer ersten Schnittansicht der zumindest zwei Schnittansichten der Körperregion (31) des Patienten (15) parallel zu einer Okklusionsebene des Patienten (15) entlang eines Abschnitts eines Zahnbogens ausgerichtet ist, und eine Bezugsebene einer zweiten Schnittansicht der zumindest zwei Schnittansichten der Körperregion (31) des Patienten (15) orthogonal zu der Bezugsebene der ersten Schnittansicht der Körperregion (31) des Patienten (15) ausgerichtet ist.

13. Eine Bildgebungsvorrichtung (1) zur Erfassung von Bilddaten (33) einer Körperregion (31) eines Patienten (15), umfassend eine Steuereinheit (22), eine Ausgabeeinheit (23; 24) und eine Benutzerschnittstelle (23; 25), wobei die Steuereinheit (22) dazu ausgebildet ist, ein Verfahren nach einem der vorhergehenden Ansprüche zu koordinieren und mittels der Bildgebungsvorrichtung (1) auszuführen, wobei die Ausgabeeinheit (23; 24) dazu ausgebildet ist, einem Nutzer der Bildgebungsvorrichtung (1) die Hilfestellung (41) zur Anpassung des Bildgebungsbereichs bereitzustellen, und wobei die Benutzerschnittstelle (23; 25) dazu ausgebildet ist, dem Nutzer ein Anpassen des Bildgebungsbereichs (35) in Abhängigkeit der Hilfestellung (41) zur Anpassung des Bildgebungsbereichs zu ermöglichen.

14. Die Bildgebungsvorrichtung nach Anspruch 13, wobei die Bildgebungsvorrichtung (1) als ein Magnetresonanzgerät (10) ausgestaltet ist.

15. Ein Computerprogrammprodukt, welches direkt in eine Speichereinheit (29) einer Recheneinheit (28) einer Bildgebungsvorrichtung (1) nach einem der Ansprüche 13 oder 14 ladbar ist, mit Programmcode-Mitteln, um ein Computer-implementiertes Verfahren nach einem der Ansprüche 1 bis 12 auszuführen, wenn das Computerprogrammprodukt in der Recheneinheit (28) der Bildgebungsvorrichtung (1) ausgeführt wird.
